# NOUVEAU FASCICULE DE BREVET EUROPEEN

(11) **EP 0 787 191 B2**
(45) Date de publication et mention de la décision concernant l'opposition: **26.10.2011**
(45) Mention de la délivrance du brevet: 26.09.2007
(21) Numéro de dépôt: 95935996.9
(22) Date de dépôt: 20.10.1995
(51) Int. Cl.: C12N 15/49, C07K 14/16, C07K 16/10, A61K 39/21

(54) **SEQUENCES NUCLEOTIDIQUES D'ANTIGENES RETROVIRAUX VIH-1 GROUPE (OU SOUS-GROUPE) O**
NUKLEOTIDEN-SEQUENZEN AUS RETROVIREN-ANTIGENEN HIV-I GRUPPE (ODER UNTERGRUPPE) O
NUCLEOTIDE SEQUENCES OF HIV-1 TYPE (OR SUBTYPE) O RETROVIRUS ANTIGENS

(30) Priorité: 20.10.1994 FR 9412554; 03.03.1995 FR 9502526
(43) Date de publication de la demande: 06.08.1997
(60) Demande divisionnaire: 07003648.8
(73) Titulaire: INSTITUT PASTEUR, 75724 Paris Cedex 15 (FR)
(72) Inventeur: CHARNEAU, Pierre, F-75005 Paris (FR); CLAVEL, François, F-75016 Paris (FR); BORMAN, Andrew, F-28210 Nongent-le-Roi (FR); QUILLENT, Caroline, F-94400 VITRY SUR SEINE (FR); GUETARD, Denise, F-75015 Paris (FR); MONTAGNIER, Luc, F-92000 Le Plessis-Robinson (FR); DONJON DE SAINT-MARTIN, Jacqueline, F-92140 Clamart (FR); COHEN, Jacques, H., M., F-51100 Reims (FR)
(74) Mandataire: Desaix, Anne
(86) Numéro de dépôt international: PCT/FR1995/001391
(87) Numéro de publication internationale: WO 1996/012809

(56) Documents cités:
- EP-A- 0 284 587
- EP-A- 0 290 893
- EP-A- 0 330 359
- EP-A- 0 471 407
- EP-A- 0 570 357
- EP-A- 0 591 914
- WO-A-90/13564
- WO-A-90/15158
- WO-A-91/09869
- WO-A-92/05266
- WO-A-94/03555
- WO-A-94/26298
- WO-A-94/28871
- WO-A-94/29339
- FR-A- 2 707 091
- US-A- 5 352 576
- VIROLOGY, vol. 205, no. 1, 15 Novembre 1994 pages 247-253, CHARNEAU, P. ET AL. 'Isolation and envelope sequence of a highly divergent HIV-1 isolata: definition of a new HIV-1 group'
- JOURNAL OF VIROLOGY, vol. 68, no. 3, Mars 1994 pages 1581-1585, GÜRTLER, L. G. ET AL. 'A new subtype of Human Immunodeficiency Virus Type 1 (MVP-5180) from Cameroon'
- AIDS, vol. 8, no. 10, 1 Octobre 1994 pages 1405-1412, NKENGASONG J.N. ET AL. 'Genomic subtypes of HIV-1 in Cameroon'
- JOURNAL OF VIROLOGY, vol. 68, no. 3, Mars 1994 pages 1586-1596, VAN DEN HAESEVELDE, M. ET AL. 'Genomic cloning and complete sequence analysis of a highly divergent african Human Immunodeficiency Virus isolate'
- THE LANCET, vol. 344, no. 8933, 12 Novembre 1994 pages 1333-1334, SCHABLE, C. ET AL. 'Sensitivity of United States HIV antibody tests for detection of HIV-1 group 0 infections'

## Description

L'invention concerne les antigènes obtenus par expression de séquences nucléotidiques ou par synthèse chimique par exemple en utilisant des synthétiseurs de marque Applied-Biosystems présents dans les variants dVIH-1 groupe (ou sous-groupe) O et particulièrement les antigènes correspondant à ceux isolables des particules virales. A titre d'exemple de virus VIH-1 du sous-groupe O, on se réfère à l'isolat VIH-1_{(VAU)} ainsi qu'à l'isolat VIH-1 _{(DUR)}.

Rappelons que la désignation HIV utilisée dans ce texte correspondant à la désignation VIH également utilisée.

L'invention concerne également les anticorps, polyclonaux ou monoclonaux, induits par ces antigènes.

L'invention est également relative à des séquences d'ADN clonées soit présentant une analogie de séquence, soit une complémentarité avec l'ARN génomique du virus précité. Elle concerne aussi des procédés de préparation de ces séquences d'ADN clonées. L'invention concerne également les polypeptides contenant des séquences d'acides aminés codés par les séquences d'ADN clonées.

De plus, l'invention concerne des applications des antigènes mentionnés précédemment à la détection in vitro chez l'homme de potentialité de certaines formes du SIDA et, en ce qui concerne certains d'entre eux, à la production de compositions immunogènes et de compositions vaccinantes contre ce rétrovirus. De même, l'invention concerne les applications aux mêmes fins des susdits anticorps et, pour certains d'entre eux, leur application à la production de principes actifs de médicaments contre ce sida humain.

L'invention concerne également l'application des séquences d'ADN clonées et de polypeptides obtenus à partir de ces séquences comme sondes ou amorces pour l'amplification génique, dans des trousses de diagnostics.

L'invention est également relative à des compositions d'antigènes, qui peuvent être obtenus par synthèse chimique ou par expression dans un hôte cellulaire recombinant et permettent le diagnostic d'une infection due à un rétrovirus humain de type HIV indépendamment du sous-type VIH-1 ou VIH-2. De telles compositions comprennent au moins un peptide choisi parmi les peptides antigéniques communs aux virus VIH-1, VIH-2, VIH-1 _{(DUR)} et VIH-1_{(VAU)} ou des variants de ces peptides antigéniques possédant des caractéristiques immunogènes semblables.

L'invention vise également des compositions permettant le diagnostic spécifique d'une infection due à un rétrovirus humain de type VIH-1, plus particulièrement le VIH-1, groupe M, le VIH-2 ou le VIH-1 groupe (ou sous-groupe) 0 et comprenant au moins un peptide antigénique spécifique du virus VIH-1, un peptide antigénique spécifique du virus VIH-2 et un peptide antigénique spécifique du virus VIH-1 groupe (ou sous-groupe) O ou des variants de ces peptides antigéniques possédant des caractéristiques immunogènes semblables. Plus particulièrement, tes peptides antigéniques sont dérivés de la protéine d'enveloppe des virus VIH-1 groupe M, VIH-2 et VIM-1 groupe (ou sous-groupe) O.

L'invention décrit par ailleurs un peptide permettant une détection d'anticorps anti-HIV que les peptides de l'art antérieur ne permettaient pas toujours de détecter en se basant notamment sur la découverte d'une nouvelle souche VIH-1: VIH-1 DUR. L'antisérum dirigé contre elle ne présente pas toujours de réactivité avec les peptides du consensus HIV tel qu'il est utilisé de nos jours. Le terme «consensus HIV » se réfère aux régions conservées entre isolats et dont la mise en évidence est essentielle à la conception de vaccins ou de réactifs diagnostiques, et dont les mutations confèrent la résistance aux médicaments antiviraux. Le terme «peptide» utilisé dans le présent texte, définit aussi bien les oligopeptides que des polypeptides.

### Etat de la technique

Deux types de virus de l'immunodéficience humaine (HIV) ayant une responsabilité dans le développement d'un SLA ou du SIDA ont été isolés et caractérisés. Un premier virus dénommé LAV-1 ou VIH-1 a été isolé et décrit dans la demande de brevet GB 8324.800 et la demande EP 84401.834 du 14/09/1984. Ce virus a également été décrit par F. Barré-Sinoussi et al dans Science (1983), 220. 868-871.

Le rétrovirus HIV de type 2 appartient à une classe distincte et n'a qu'une parenté immunologique réduite avec des rétrovirus HIV de type 1. Les rétrovirus VIH-2 ont été décrits dans la demande de brevet européen n° 87.400.151.4 publiée sous le numéro 239.425.

Le rétrovirus VIH-1 est le plus courant et sa présence est prédominante dans plusieurs régions du monde. Quant au rétrovirus VIH-2, il est le plus souvent retrouvé en Afrique de l'Ouest, bien que sa propagation à l'extérieur de cette région ait été récemment documentée par Grez et al, (1994) J. Virol. 68, 2161-2168.

L'ensemble des lentivirus de l'immunodéficience des primates, comprenant les virus du type 1 et du type 2 de l'immunodéficience humaine ainsi que plusieurs types de virus de primates non humains, augmente en taille et en complexité. Le plus courant de ces virus, le VIH-1, se répand actuellement sous forme d'épidémie mondiale et il est responsable d'un problème de santé public majeur. Peu après l'identification et la caractérisation moléculaire de ce virus, il a été reconnu comme étant très variable, et comprend actuellement plusieurs sous-types (Myers, 1994, Louwagie, et al. 1993, Louwagie, et al. 1992, Myers, G. (1994) VIH-1 subtypes and phylogenetics trees. In: Human Retrovirus and AIDS 1994; Myers, G., Korber, B., Wain-Hobson, S., Smith, R.F. and Pavlakis, G.N., eds. Los Alamos National Laboratory, Los Alamos, NM. III-2 - III-9.). Cette distinction de sous-types est surtout basée sur la divergence des gènes *gag* et *env.* Au moins 6 sous-types ont été identifiés, désignés A à F, mais plusieurs sont encore susceptibles d'émerger de l'enquête mondiale poussée en cours sur les isolats du VIH-1. Il a été trouvé que ces différents sous-types sont équidistants les uns des autres, dans un profil phylogénétique dit phylogénie en étoile, ce qui suggère que les différents sous-types du VIH-1 auraient évolué et divergé de façon synchrone à partir d'un ancêtre commun.

Récemment, deux virus distincts de ce groupe de virus VIH-1 ont été isolés et caractérisés. Ces deux virus ont été obtenus de patients vivant au Cameroun, en Afrique de l'Ouest Centrale (Gürtler, et al. 1994, Vanden Heasevelde, et al. 1994). Leur séquence, plus particulièrement la séquence de leur gène env (enveloppe), montre clairement que ces virus appartenaient à une catégorie distincte de virus apparentés au VIH-1, désignée VIH-1 groupe O (NKENGASONG et al., 1993).

Toutefois, la diversité des isolats à l'intérieur de ce groupe de virus apparentés au VIH-1 n'est pas connue, et sa propagation en dehors de l'Afrique n'a pas été documentée.

Une contrainte générale, dans la mise au point de tests sérologiques HIV, est d'éviter aussi bien les réactions faussement positives - ou faussement négatives - tout en conservant ou améliorant la sensibilité que permettent les tests antérieurs en détection de séropositivité.

Les tests basés sur l'emploi de peptide(s) consensus, essentiellement dérivés du gène « *env »,* ont été considérés comme une solution presque idéale jusqu'à ce que la découverte du variant VIH-1-O fasse entrevoir la possibilité de résultats faussement négatifs (Genomic cloning and complete séquence analysis of a highly divergent African human immuno-deficiency virus isolate. J. Virol. 1994; 68: 1586-96; a new subtype of human immuno-deficiency virus type 1 (MPV-51 00) from Cameroon. J. Virol. 1994; 68:1581-85).

La non-réactivfté de certains tests à antigène peptidique *« env*», chez des patients présentant quand même certains syndromes cliniques caractéristiques du SIDA ou des syndromes lymphadénopathiques qui parfois les précèdent, est. à ce jour, parfois imputée à une infection du groupe VIH-1-O (VIH-1/VIH-2 seronegativity in VIH-1 subtype O infected patients, Lancet 1994; 343:1393-94 : New VIH-1 subtype in Switzerland. Lancet 1994; 344:270-71).

### Description de l'invention

La présente invention a pour but de mettre à disposition des laboratoires de diagnostic des moyens, notamment des peptides spécifiques permettant une détection d'anticorps anti-HIV, qui étaient jusqu'à ce jour susceptibles d'être indétectables. La présente demande décrit également des mélanges de peptides issus de VIH-1 DUR et de peptides correspondants d'autres HIVs, de façon à éviter les résultats « faux négatifs » potentiels.

Elle concerne par ailleurs un procédé de détection et de discrimination dans un échantillon biologique entre des anticorps caractéristiques d'un rétrovirus du type VIH-1-M et des anticorps caractéristiques d'un rétrovirus VIH-1 groupe (ou sous-goupe) O.

L'invention découle des observations faites sur une femme séropositive, ayant séjourné au Cameroun et ayant révélé une réactivité sérologique atypique, au cours de plusieurs tests de dépistages de l'infection HIV, ces derniers ayant été confirmés par des techniques de « Western blot ».

Du fait de cette réactivité sérologique atypique, en particulier du manque de réactivité sur certains tests de troisième génération, même modifiés pour le type 0, les inventeurs ont jugé intéressant d'entreprendre un séquencage de certaines parties du génome de cette souche VIH-1 DUR, plus spécifiquement des gènes GAG et ENV.

Cependant, les amplifications géniques par PCR à l'aide d'amorces issues du groupe M et d'amorces connues du groupe O ont été sans résultat pour les parties codant la boucle V3 de gp120, et pour la région immunodominante de gp41. Seule la région GAG était amplifiable à l'aide d'amorces connues dans l'art antérieur (Loussert-Ajaka l, Lancet 1994; 343:1393). Un autre but de la présente invention est par conséquent la détermination d'amorces susceptibles de palier ce problème. Ces amorces pourront indifféremment être dénommées « primers » par la suite.

On a déterminé des séquences partielles des glycoprotéines gp41 et de gp120 ainsi que de protéines de capside (gène GAG) à partir d'ADN lymphocytaire et de cultures virales, indiquant que cette souche VIH-1 DUR appartient d'une part au groupe VIH-1-O, et qu'elle diffère de façon importante du groupe M, plus particulièrement pour gp41 et gp120.

Ainsi, on a pu mettre en évidence, plus particulièrement à propos de la séquence GAG de VIH-1 _{(DUR)}, l'existence de séquences consensus dans le groupe O , sur plusieurs zones, distinctes de séquences consensus du groupe M sur les mêmes zones.

Le clonage des séquences codant les fragments de GAG, gp41 et gp120 de VIH-1 _{(DUR)} a été effectué dans un plasmide Bluescript® contenant un site PST1. Les produits d'amplification ont été clonés soit selon les techniques classiques utilisant les amorces universelles T3 et T7, soit directement séquencés par l'utilisation des amorces de la précédente amplification. Les séquences ont ensuite été déterminées par le dispositif automatique de séquençage Applied Biosystems 373A (ESGS Montigny le Bretonneux, France).

Dans le contexte de la présente invention, les inventeurs ont isolé et séquencé le gène *env* d'un isolat du groupe O, le VIH-1_{(VAU)}, obtenu d'une patiente française qui n'a jamais voyagé en dehors de l'Europe et qui est morte du SIDA en 1992. Selon sa séquence d'enveloppe, le VIH-1_{(VAU)} est apparenté aux deux virus camerounais HIV_{ANT70} et HIV_{MVP5180} récemment caractérisés. L'analyse phylogénétique des séquences *env* révèle que les trois virus semblent constituer un groupe distinct, qui sera appelé ici VIH-1 groupe O. L'isolement du VIH-1_{(VAU)} de cette patiente indique également qu'une certaine propagation des VIH-1 du groupe O s'est déjà produite en dehors de l'Afrique.

### Isolement du virus VIH-1_{(VAU)}

VIH-1_{(VAU)} a été isolé en 1992 d'une patiente française âgée de 41 ans atteinte du SIDA. Cette patiente présentait en 1986 une leuco-neutropénie sévère associée à un carcinome du col utérin. Néanmoins, elle a progressivement montré des signes d'infections opportunistes, avec une baisse du nombre de cellules T CD4⁺ circulantes et elle est morte du SIDA en 1992. Des anticorps anti-VIH-1 ont d'abord été détectés par ELISA (Elavia, SANOFI DIAGNOSTICS PASTEUR, et Test ABBOTT) en 1990.

La patiente n'avait jamais voyagé en dehors de l'Europe, n'avait pas utilisé de médicaments par voie intraveineuse et n'avait subi aucune transfusion sanguine connue. Aucun partenaire sexuel originaire de l'Afrique n'a été identifié. Elle a donné naissance à un enfant en bonne santé en 1971, mais un fils, né en 1980, est mort à l'âge d'un an suite à un épisode clinique très suggestif de SIDA néo-natal. Son troisième enfant né en 1983 et son mari sont actuellement en bonne santé et non infectés.

L'isolement du virus a été réalisé de la manière suivante : les cellules CD8+ présentes dans les PBMC (lymphocytes sanguins périphériques) de la patiente ont été éliminées à l'aide de billes revêtues d'anticorps IOT8 (Immunotech). Ces PBMC restantes ont été stimulées à la PHA, puis cocultivées avec des PBMC épuisées en CD8 provenant d'un donneur sain et stimulées à la PHA. La croissance virale dans la coculture a été suivie par dosage de l'activité transcriptase inverse (RT) du surnageant et par test ELISA de la p24 du VIH-1 (coffret de diagnostic commercialisé par DuPont de Nemours). Le virus obtenu de la coculture initiale a été soumis à plusieurs passages dans des cultures de PBMC épuisées en CD8 et stimulées à la PHA. Plusieurs tentatives ont été réalisées pour infecter par le VIH-1_{(VAU)} diverses lignées cellulaires transformées, dont des cellules MT4 (Harada, et al. 1985) et CEM (Rey, et al. 1989), ainsi que la lignée P4-2 de cellules Hela-CD4-LTRLacZ (Clavel et Chameau 1994).

### Caractérisation biologique du VIH-1_{(VAU)}

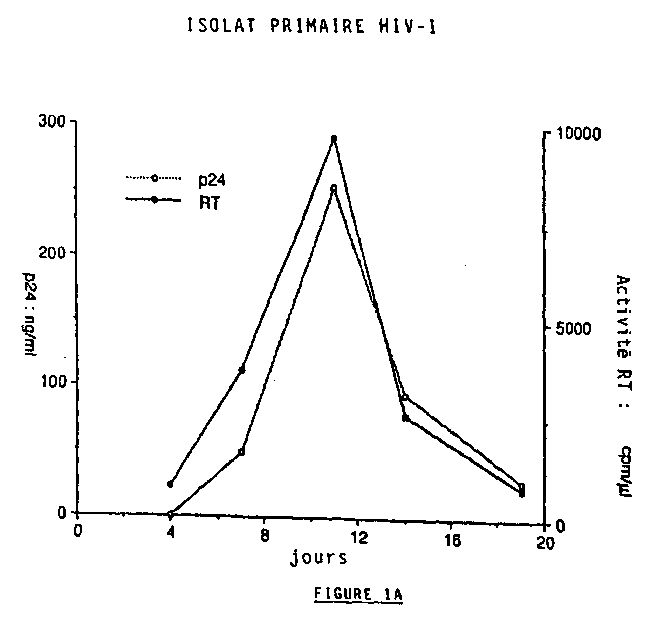

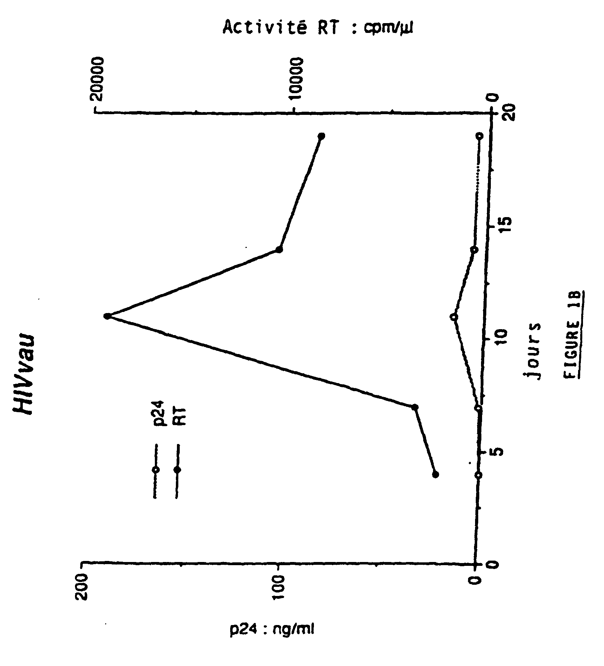

Deux semaines après la coculture des PBMC de la patiente épuisées en CD8, stimulées à la PHA, avec des cellules similaires d'un donneur sain, la production de virus a été détectée sous la forme d'un pic d'activité RT dans le surnageant de culture. Ce virus pouvait alors être soumis à des passages en série sur des PBMC normales, épuisées en CD8, stimulées à la PHA Sur la figure 1, le panneau A représente la production du VIH-1_{(VAU)} dans des sumageants de cultures de PBMC infectées, contrôlées par dosage RT (cercles pleins) et ELISA de capture d'antigène p24 VIH-1 (cercles vides). La concentration de p24 VIH-1 est exprimée en ng/ml et l'activité RT en cpm/µl. Dans le panneau B, la même expérience a été effectuée avec un isolat primaire standard du VIH-1 d'un patient atteint du SIDA

Bien que la croissance du VIH-1_{(VAU)} ait été facilement détectée à l'aide du dosage RT, la détection de virus dans les surnageants de culture à l'aide de l'ELISA p24 VIH-1 (DuPont) était nettement moins sensible. La figure 1 montre la comparaison entre les profils d'infection productive des PBMC avec soit le VIH-1_{(VAU)} soit un isolat primaire du VIH-1 d'un patient atteint du SIDA, dosés en RT ou p24. Pour des quantités équivalentes de particules, déterminées par dosage de l'activité RT dans les sumageants dosés, approximativement 25 fois moins de p24 a été détecté pour le VIH-1_{(VAU)} que pour l'autre isolat VIH-1. La différence peut être consécutive au fait que l'anticorps monoclonal spécifique de la p24 du VIH-1, qui est utilisé pour revêtir les plaques ELISA, n'a qu'une faible affinité pour les produits gag du VIH-1_{(VAU)}.

Plusieurs tentatives négatives ont été faites pour propager le VIH-1_{(VAU)} sur des lignées de cellules T humaines transformées, sensibles au VIH-1. En particulier, des cocultures entre des PBMC infectées par le VIH-1(VAU) et soit des cellules MT4 soit des cellules CEM n'ont pas conduit à la propagation du virus. Il a également été trouvé que ce virus n'était pas capable d'infecter des cellules HeLa CD4⁺ (P4-2) (Clavel et Chameau 1994) portant un gène lacZ inductible par le gène tat. De même, aucune réplication du VIH-1_{(VAU)} ne pouvait être détectée dans des lymphocytes sanguins périphériques activés de plusieurs chimpanzés.

L'analyse de la séquence d'enveloppe VIH-1_{(VAU)}, qui sera décrite en détail plus loin, et sa comparaison avec celle des deux isolats camerounais récemment décrits indiquent que tous les trois virus appartiennent au même groupe de virus apparentés au VIH-1. De plus, cette comparaison indique que ces trois variantes du virus sont approximativement phylogénétiquement équidistantes les unes des autres. Par conséquent, chacune des trois variantes de virus constitue en soi un sous-type, distinct, de leur groupe, qui est maintenant appelé VIH-1 groupe O. Ce groupe est distinct du groupe des autres isolats VIH-1, identifiés jusqu'ici, que les inventeurs appellent ici VIH-1, groupe M.

L'apparition de ce nouveau groupe pose la question de son origine : est-ce que le groupe O a évolué à partir des virus du groupe M (ou inversement) ou est-ce que chaque groupe a un passé distinct ? Les inventeurs pensent que dans la mesure où à la fois le groupe M et le groupe O présentent un profil de divergence interne similaire, il est vraisemblable qu'ils correspondent chacun à la diversification d'ancêtres distincts de virus dans des populations humaines distinctes. On ne peut pas apprécier à partir des données phylogénétiques et virologiques actuellement disponibles si l'ancêtre de l'un ou l'autre des deux groupes affectait les humains de manière naturelle ou a été introduit chez les humains à partir d'autres espèces. Le seul virus proche du VIH-1 présent chez un primate non humain est l'isolat SNCPZGAB (Huet, et al. 1990), isolé à partir d'un chimpanzé apparemment infecté de façon naturelle, qui est clairement distinct à la fois du groupe M et du groupe O, et pour lequel aucun équivalent humain n'a été trouvé, Il est peu probable que les virus du groupe O aient récemment évolué à partir d'un virus de chimpanzé dans la mesure où le VIH-1 _{(VAU)} n'a pas réussi à se répliquer dans des lymphocytes de chimpanzés.

Pourquoi l'épidémie du groupe O n'apparaît-elle que maintenant, quelques 15 à 20 ans plus tard que le groupe M ? Il y a trois explications possibles: premièrement, l'introduction de l'ancêtre des virus du groupe O chez les humains se serait produite plus récemment que celle du groupe M; deuxièmement, il se pourrait qu'on ait permis au groupe M de se propager plus tôt que le groupe O en raison de différentes conditions sociales dans leur région d'origine; et troisièmement, les virus du groupe O auraient une capacité de transmission plus réduite, comparée à celle des virus du groupe M. Il a été proposé qu'une telle propriété explique l'absence de propagation mondiale significative du VIH-2, pour lequel une charge virale plus faible chez les sujets infectés est liée à une transmissibilité réduite (De Cock, et al. 1993). A cet égard, bien que nous n'ayons aucune donnée sur la charge virale chez les patients infectés par un VIH-1 du groupe O, le pouvoir pathogène de ces virus ne semble pas être différent de celui du VIH-1. La patiente chez qui le VIH-1_{(VAU)} a été isolé est morte du SIDA, comme le patient chez qui l'isolat HIV_{MVP5180} du groupe O a été obtenu.

Toutefois, l'histoire naturelle de l'infection de la patiente VIH-1 _{(VAU)} n'est toujours pas claire, mais il existe plusieurs indications que cette patiente a été infectée avant 1980, comme le suggère la mort à cette date de son deuxième enfant atteint d'un syndrome ressemblant au SIDA.

La présente invention est également relative à des compositions contenant soit des antigènes selon l'invention, soit un mélange d'antigènes selon l'invention associés à des extraits originaires d'un ou plusieurs VIH-1 groupe O ou d'autres virus variants, d'une part, et d'un ou plusieurs VIH-2 et/ou VIH-1, d'autre part, ces compositions étant éventuellement marquées. On peut utiliser tous types de marqueur approprié : enzymatiques, fluorescents, radioactifs, etc.

### Acides nucléiques

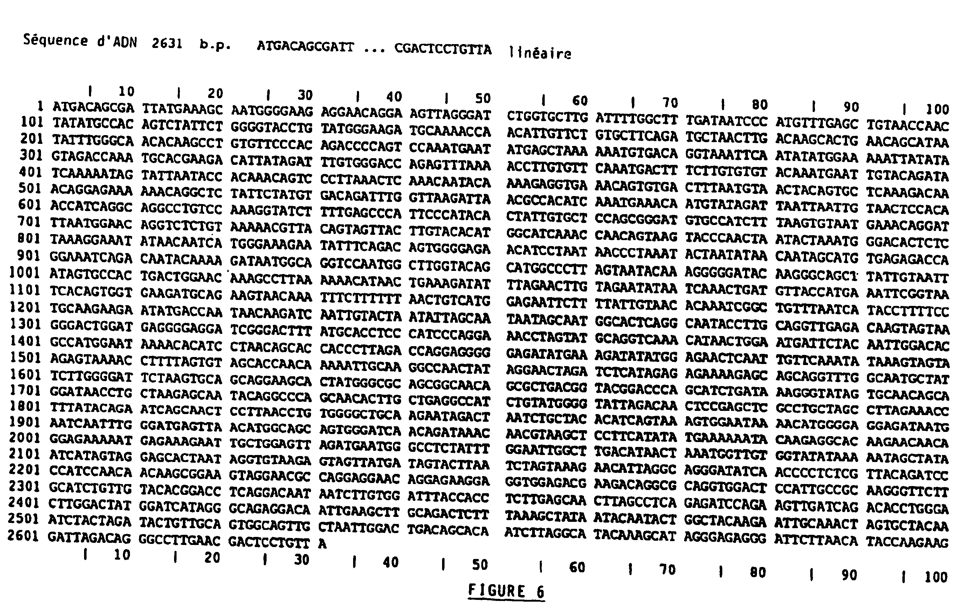

L'invention concerne les ADN ou fragments d'ADN, plus particulièrement ADN et fragments d'ADN clonés obtenus à partir de l'ARN, d'ADNc ou d'amorces utilisables en PCR, ou autres méthodes d'amplification génique dérivés de l'ARN ou de l'ADN du rétrovirus VIH-1_{(VAU)}. La demande décrit également des ADN équivalents, notamment présentant des homologies de séquence avec l'ADN du VIH-1 _{(VAU)}, en particulier avec la séquence codant la région *env* de la souche VIH-1_{(VAU)} comprenant la séquence représentée à la figure 6 et appelée « vau ». L'homologie avec le VIH-1 groupe M est au moins égale à 50%, de préférence à 70% et plus avantageusement encore à environ 90%. D'une façon générale, la demande décrit tout ADN (ou ARN) équivalent capable de s'hybrider avec l'ADN ou l'ARN d'un rétrovirus VIH-1 du groupe O.

L'invention concerne aussi les séquences d'ARN correspondant aux séquences d'ADN définies ci-dessus.

L'invention a aussi pour objet des compositions contenant les peptides ou polypeptides codés par l'ADN susdite ou des fragments d'ADN.

Des oligonucléotides dérivés de la séquence VAU particulièrement des oligonucléotides comprenant au moins 9 nucléotides, peuvent être utilisés pour la détection de séquences ADN ou ARN de virus VIH-1 du groupe O dans des prélèvements biologiques, des cultures cellulaires, ou des extraits de cellules, par la technique de la PCR ou toute autre technique d'amplification génique. Ces séquences pourraient être utilisées soit comme amorces d'amplification génique soit comme sondes en vue de la détection spécifique des produits d'amplification génique. Sont utilisables également comme sonde d'hybridation, les produits d'amplification, ou leur séquence correspondante synthétique, obtenus par synthèse chimique (Applied Biosystems).

L'invention couvre également tout fragment d'au moins 100 nucléotides utilisable comme sonde dans des réactions d'hybridation et capable de permettre une réaction avec une partie du génome d'un variant VIH-1_{(VAU)} dans des conditions d'hybridation de forte stringence.

### Clonage et séquencage du gène env VIH-1_{(VAU)}

Pour l'amplification initiale par PCR de l'ADN du VIH-1_{(VAU)}, l'ADN total a été extrait de PBMC infectées par le VIH-1_{(VAU)} et un segment du gène pol (région intégrase) a été amplifié à l'aide d'amorces dégénérées :
amorce 4506 : 5'AGTGGAT(A/T)(T/C)ATAGAAGCAGAAGT3'; Seq. ID N°1;
amorce 5011 : 5'ACTGC(C/T)CCTTC(A/C/T)CCTTTCCA3'; Seq. ID N°2;

Le milieu de réaction incluant KCl 50 mM, Tris-HCl 10 mM (pH 8,9), MgCl₂ 1,5 mM, gélatine 0,1 mg/ml, dNTP 0,2 mM, Taq Polymérase 1 U (Amersham). La PCR a été effectuée pendant 43 cycles thermiques à 92°C pendant 10 secondes, 50°C pendant 1 minute, 72°C pendant 40 secondes.

Le produit d'amplification résultant a été cloné dans un vecteur pBluescript, engendrant le clone ph4 déposé à la CNCM le 20 octobre 1994 sous le n° I-1486 qui a ultérieurement été utilisé comme sonde pour cribler une banque lambda d'ADN de faible poids moléculaire, digéré par EcoRI, issu de cellules infectées par le VIH-1_{(VAU)}. Brièvement, les PBMC infectées par le VIH-1_{(VAU)} ont été cocultivées pendant 24 heures avec des PBMC neuves stimulées à la PHA et épuisées en cellules CD8⁺, après quoi un effet cytopathogène poussé (ECP) était visible. L'ADN de faible poids moléculaire a alors été extrait suivant la méthode de Hirt (Hirt 1967), et digéré avec l'enzyme EcoRl. Une analyse précédente en Southern-blot précédente de cet ADN avait bien montré que le génome VIH-1_{(VAU)} contenait un seul site EcoRl, permettant le clonage d'espèces d'ADN circulaire non intégré représentant la totalité du génome viral. Le produit de digestion résultant a été soumis à une électrophorèse sur gel d'agarose, et la population de fragments d'ADN d'une taille de 8-12 kb approximativement a été purifiée et ligaturée à de l'ADN lambda Zap digéré par EcoRl (Stratagene). Après l'encapsidation, l'étalement et le criblage par hybridation avec de l'ADN ph4 marqué au ³²P, un clone, λ H34, a été identifié comme étant positif, et amplifié. L'inserat EcoRI a été purifié, soniqué, et cloné par "shotgun" dans le vecteur M13mp18 traité à la phosphatase, digéré avec l'enzyme SmaI. Cent cinquante des clones obtenus ont été séquencés sur un séquenceur d'ADN 373A (Applied Biosystems), et les séquences résultantes ont été rassemblées en une seule séquence à l'aide du progiciel d'analyse d'ADN GCG-Wisconsin.

L'analyse de cette séquence a révélé de nombreux codons non-sens dans tous les cadres de lecture, ce qui est très suggestif d'un génome hypermuté (Vartanian, et al. 1991). Cette séquence étant inutilisable, il a par conséquent été décidé d'amplifier par la PCR le gène *env* du VIH-1_{(VAU)} à l'aide de l'ADN total de PBMC infectées par le VIH-1_{(VAU)}, et des amorces oligonucléotidiques dérivées de la séquence λH34:
amorce TH2 5'GCTCTAGATGGGGATCTCCCATGGCAGG3' Seq. ID N° 3;
amorce UH2 5'GCTCTAGATCAGGGAAGAATCCCTGAGTGT3'. Seq. ID N° 4.

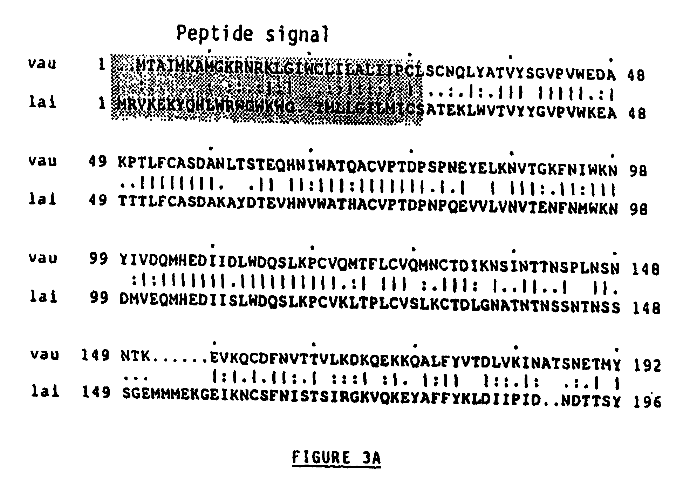

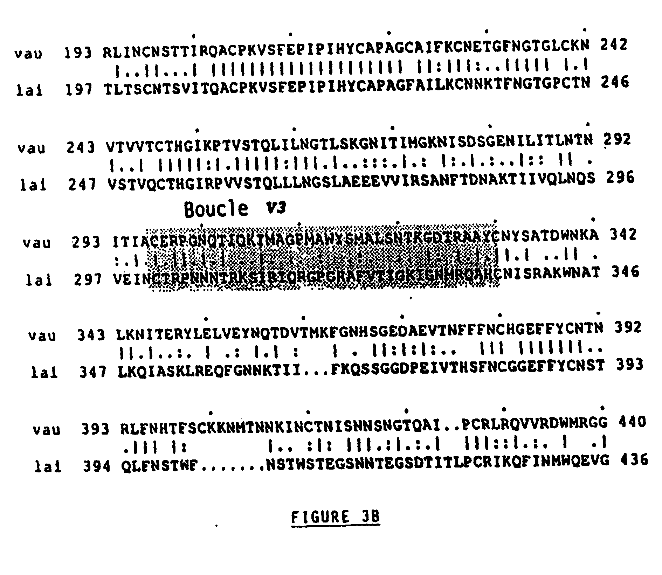

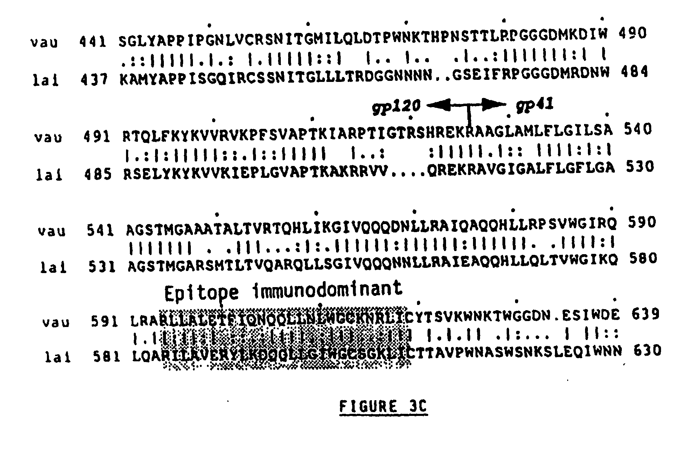

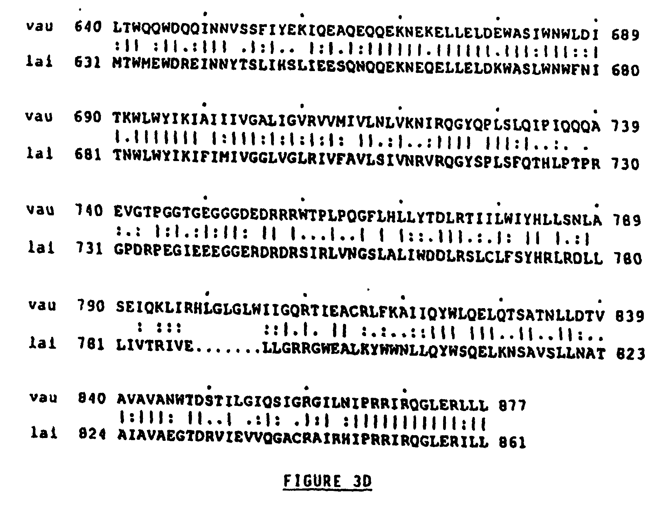

L'amplification par la PCR a été effectuée pendant 35 cycles thermiques à 92°C pendant 15 secondes, 52°C pendant 1 minute, 60°C pendant 2 minutes et 72°C pendant 2 minutes. Le produit d'amplification résultant, d'une taille de 3,5 kb, a été cloné dans le vecteur M13mp18 et séquence par réactions successives, en utilisant d'abord l'amorce universelle de séquençage M13, puis des amorces déduites des séquences amont. L'analyse des séquences nucléotidiques et peptidiques a été effectuée à l'aide du progiciel d'analyse d'ADN GCG-Wisconsin. Le gène *env* VIH-1_{(VAU)} code 877 acides aminés au total, incluant le peptide signal. La séquence nucléotidique du gène *env* VIH-1_{(VAU)} correspond à la Seq. ID N° 5 (voir figure 3).

### Utilisation d'acides nucléiques à titre de sondes

L'invention concerne naturellement aussi l'utilisation des ADN, ADN_{c} ou de leurs fragments, ou de plasmides recombinants ou autres vecteurs équivalents contenant ces fragments, en tant que sondes, pour la détection de la présence ou non du virus VIH-1(_{VAU}) dans des échantillons de sérum ou autres liquides ou tissus biologiques obtenus à partir de patients suspectés d'être porteurs du virus VIH-1(_{VAU}). Ces sondes sont éventuellement marquées (marqueurs radioactifs, enzymatiques, fluorescents, etc.). Des sondes particulièrement intéressantes pour la mise en oeuvre du procédé de détection du virus VIH-1_{(VAU)} ou d'un variant VIH-¹_{(VAU)} peuvent être caractérisées en ce qu'elles comprennent la totalité ou une fraction de l'ADN complémentaire du génome du virus VIH-1_{(VAU)} ou encore notamment les fragments contenus dans divers clones. Des sondes caractérisées en ce qu'elles comprennent une fraction de l'ADNc du VIH-1_{(VAU)} contenant tout ou partie de la région *env.* font partie de l'invention.

Les sondes mises en oeuvre dans ce procédé de détection du virus VIH-1_{(VAU)} ou dans des trousses de diagnostic, ne sont en aucune façon limitées aux sondes décrites précédemment. Elles comprennent toutes les séquences nucléotidiques issues du génome du virus VIH-1_{(VAU)}, d'un variant VIH-1_{(VAU)} ou d'un virus proche par sa structure, dès lors qu'elles permettent la détection, à partir de fluides biologiques de personnes susceptibles de présenter un sida, d'un virus VIH-1 du groupe O, en particulier VIH-1_{(VAU)} par hybridation avec de l'ADN ou de l'ARN du virus VIH-1_{(VAU)}.

Particulièrement avantageuses sont les sondes qui, lors de l'hybridation avec VIH-1, donnent une réaction forte avec les HIV appartenant au groupe O et une réaction faible avec les VIH-1 appartenant au groupe M.

La détection peut être réalisée de toute façon en soi connue, notamment :
par une mise en contact de ces sondes soit avec les acides nucléiques obtenus à partir de cellules contenues dans des fluides biologiques (par exemple liquide céphalo-rachidien, salive, etc.), soit avec ces fluides eux-mêmes, dès lors que leurs acides nucléiques ont été rendus accessibles à l'hybridation avec ces sondes, et ce dans des conditions permettant l'hybridation entre ces sondes et ces acides nucléiques
et par une détection de l'hybridation éventuellement produite.

Le susdit diagnostic mettant en jeu des réactions d'hybridation peut également être réalisé à l'aide de mélanges de sondes respectivement dérivées du VIH-1_{(VAU)}, du VIH-1 et du VIH-2, dès lors qu'il n'est pas nécessaire de faire la distinction entre les types de virus HIV recherchés.

La demande décrit aussi des vecteurs d'expression contenant la séquence codant pour les protéines d'enveloppe de VIH-1.

L'invention comprend des compositions pour la détection de la présence ou non de virus VIH-1_{VAU} dans des échantillons de sérum ou d'autres liquides ou tissus biologiques, obtenus à partir de patients susceptibles d'être porteurs du virus VIH-1_{VAU}. Ces compositions sont caractérisées en ce qu'elles comprennent au moins une sonde obtenue à partir d'une séquence nucléotidique issue ou dérivée du génome du virus VIH-1_{VAU}, particulièrement un fragment d'ADN VIH-1_{VAU} contenant la région ou une partie de la région codant la protéine *env* du virus VIH-1_{VAU} ou d'un variant de VIH-1_{VAU}.

Avantageusement, la composition décrite ci-dessus comprend également une sonde obtenue à partir d'une séquence nucléotidique issue de VIH-1 ou de VIH-2.

D'autres compositions de diagnostic comprennent les amorces de l'invention utilisables en amplification génique de rétrovirus de sous-groupe O ou variants de ces rétrovirus.

### Antigènes, notamment protéines et glycoprotéines

L'invention concerne une protéine de rétrovirus VIH-1 groupe (ou sous-groupe) O, ou polypeptide ou peptide naturel ou synthétique comprenant au moins une partie de ladite protéine, susceptibles d'être reconnus par des anticorps isolables à partir de sérum obtenu suite à une infection par une souche VIH-1 groupe O VAU.

L'invention concerne une protéine d'enveloppe externe du rétrovirus VIH-1_{VAU} codée par le gène comprenant , la séquence représentée à la figure 6. Selon un mode de réalisation préféré de l'invention, cette protéine est en outre caractérisée en ce qu'elle comprend la séquence d'acides aminés représentée sur la figure 3 et comportant les résidus d'acides aminés 1 à 526. L'invention a aussi pour objet tout polypeptide ou variant, dérivé de ladite séquence, ayant un épitope susceptible d'être reconnu par des anticorps induits par le virus VIH-1_{VAU}.

La susdite protéine peut être obtenue sous forme glycosylée ou non glycosylée.

L'invention a aussi pour objet une protéine transmembranaire d'enveloppe comprenant l'enchaînement d'acides aminés représenté sur la figure 3 entre les résidus d'acides aminés 527 et 877. Cette protéine transmembranaire est, dans le cadre de l'invention, sous forme glycosylée ou non.

L'invention concerne tous les antigènes, notamment protéines, glycoprotéines, polypeptides ou peptides, obtenus par expression de séquences codantes du génome VIH-1_{(VAU)} et ayant des propriétés immunologiques équivalentes à ceux ou celles du VIH-1_{(VAU)}. Les antigènes sont dits équivalents dans le cadre de la présente invention, dès lors qu'ils sont reconnaissables par les mêmes anticorps, notamment des anticorps isolables à partir de sérum obtenu d'un patient ayant été infecté par un VIH-1_{(VAU)}.

En particulier, l'invention a pour objet les peptides ou polypeptides synthétisés chimiquement et dont la séquence d'acides aminés est contenue dans celle des protéines d'enveloppe VIH-1_{(VAU)}, représentée sur la figure 3, ou les peptides ou polypeptides équivalents.

On doit aussi inclure, parmi les peptides, polypeptides, protéines ou glycoprotéines équivalents, les fragments des antigènes qui précèdent et les peptides préparés par synthèse chimique ou par génie génétique, dès lors qu'ils donnent lieu à des réactions immunologiques croisées avec les antigènes dont ils sont dérivés. En d'autres termes, l'invention concerne tout peptide ou polypeptide ayant des épitopes identiques ou semblables aux épitopes des susdits antigènes, susceptibles d'être reconnus par les mêmes anticorps. Font partie de ce dernier type de polypeptides, les produits d'expression de séquences d'ADN correspondant aux séquences des ADN codant les polypeptides ou antigènes susdits.

Plus particulièrement, les antigènes obtenus à partir du virus VIH-1_{(VAU)} ou produits par génie génétique ou synthèse chimique classique et qui présentent le plus grand intérêt dans le contexte de la présente invention sont les antigènes qui permettent d'établir une distinction claire entre les virus VIH-1_{(VAU)} de l'invention et les virus des groupes VIH-1 et VIH-2. A cet égard, des différences considérables ont été observées au niveau de la protéine d'enveloppe du virus VIH-1_{(VAU)} ainsi qu'au niveau de l'épitope immunodominant de la portion externe de la protéine PM. Il semble que les protéines gag et pol présentent davantage de similitude avec le virus VIH-1 que la protéine d'enveloppe.

L'invention concerne aussi des peptides ou polypeptides identiques à la région immunodominante de la glycoprotéine transmembranaire d'enveloppe du VIH-1_{(VAU)} Cette région est représentée à la figure 3.

Des polypeptides préférés de cette région sont par exemple ceux qui contiennent la séquence CKNRLIC ou correspondent à cette séquence. Il peut s'agir aussi de polypeptides ou peptides répondant à la séquence RLLALETFIQNWWLLNLWGCKNRLIC ou comprenant cette séquence.

Un autre peptide préféré, identifié ci-après par la dénomination «peptide VAU», répond à la séquence suivante ou comprend cette séquence ou toute partie de cette séquence susceptible d'être reconnue par des anticorps dirigés contre le rétrovirus VIH-1_{(VAU)} RARLLALETFIQNQQLLNLWGCKNRLICYTSVKWNKT.

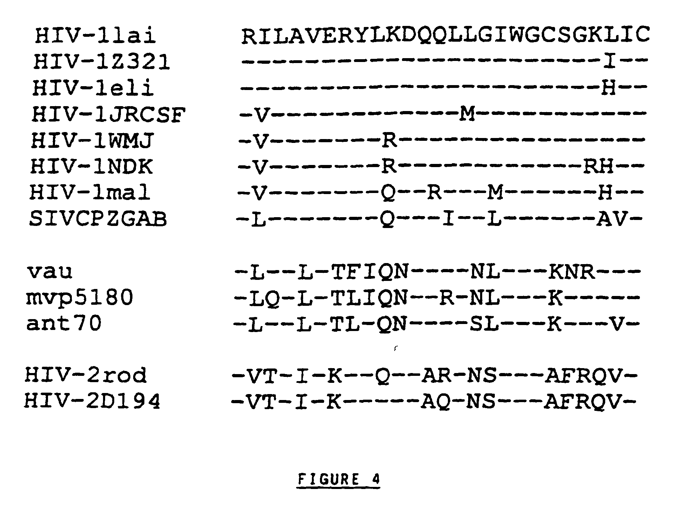

Des polypeptides variants de cette séquence sont par exemple les polypeptides représentés sur la figure 4 pour les isolats VIH-1_{MVP5180} et VIH-1_{(AN170).} Ces polypeptides peuvent aussi être dérivés des précédents par insertion et/ou délétion et/ou substitution, par exemple substitution conservative par des résidus d'acides aminés.

### Synthèse de peptides VAU

Un peptide VAU a été préparé par la technique classique de synthèse peptidique en phase solide par la méthode Fmoc en " flux continu ». Le peptide a été préparé à l'aide d'un synthétiseur Milligen 9050 PEP en utilisant la résine "Millipore" PEG PAL, substituée par le premier résidu d'acide aminé C-terminal. Les chaines latérales des acides aminés sont protégées par les groupes suivants : Pmc pour l'arginine : Trt pour l'asparagine, la glutamine et la cystéine : Boc pour la lysine ; tBu ester pour l'acide glutamique ; tBu éther pour la sérine, la thréonine et la tyrosine. Les groupements temporaires Fmoc sont éliminés par une solution de pipéridine à 20% en DMF. Les réactions de couplage de chaque acide aminé sont réalisées avec 6 équivalents de DIPCDI et d'HOBT. Certains résidus nécessitent un double couplage notamment les arginines 1 et 23, les cystéines 19 et 26, l'asparagine 11. les glutamines 10, 12 et 13, l'alanine 4, l'isoleucine 9, les leucines 2, 3, 14 et 15.

Après couplage, la résine est séchée sous vide. Le peptide est clivé du support par le réactif K pendant 4 heures à température ambiante. Le peptide brut est précipité et lavé à l'éther éthylique. Le produit est purifié par chromatographie liquide sous haute pression (HPLC) sur un instrument WATERS LC PREP 4000 avec des cartouches WATERS Delta Pak C18 40x100mm, débit 30 ml/mn, gradient acétonitrile/TFA 0,1%. Les fractions contenant le peptide sont rassemblées, concentrées à l'évaporateur rotatif puis lyophilisées.

### Cyclisation

Le peptide (0,025mM) est dissous dans une solution d'acétate d'ammonium 10mM. Le pH est amené à 8,5 avec une solution d'ammoniaque 1 M. Le pH est réajusté après 3 ou 4 heures. La cyclisation est suivie par HPLC à 214 nm et 280 nm, colonne WATERS Delta Pak C18 5µ, gradient acétonitrile/TFA 0,1%. La cyclisation est terminée au bout de 15 heures. Le pH est amené à 6 à l'aide d'acide acétique 97-100%, la solution est lyophilisée puis purifiée dans les mêmes conditions que pour le peptide brut.

Le peptide est contrôlé par HPLC et par spectrométrie de masse selon la technique d'électrospray (spectrophotomètre FISON VG Trio 2000).
Fmoc : Fluoroenyl-9 methyloxycarbonyl
Pmc : Pentanemethyl-8 chromane sulfonyl-6
Trt : Tritryl
Boc : Tertbutyloxycarbonyl
tBU : tert butyl
DMF : Dimethylformamide
DIPCDI : Diisopropyl carbodiimide
HOBT : Hydroxy-1 benzotriazole
TFA : acide trifluoroacétique
Réactif K : Phénol/Eau/Thioanisole/Ethanedithiol/TFA;
2,5ml/2,5ml/2,5ml/1,5ml/41ml
Comparaison de la séquence d'acides aminés de l'enveloppe VIH-1_{(VAU)} avec la séquence correspondante d'autres virus HIV.

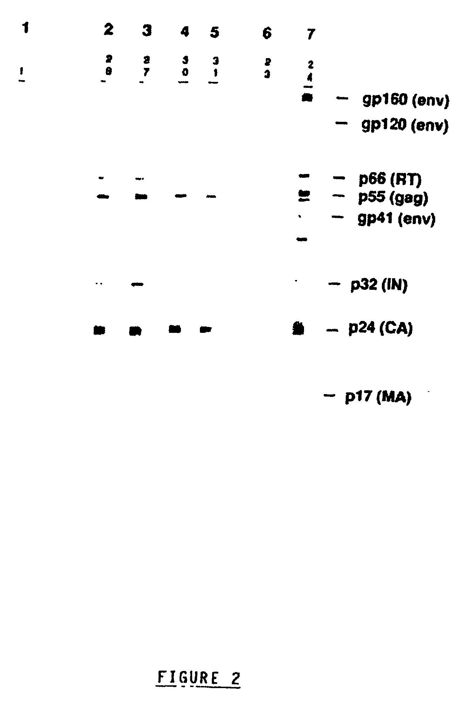

L'analyse en Western-blot d'une série d'échantillons sériques provenant d'une patiente infectée par le VIH-1(_{VAU}) est présentée sur la figure 2. Des bandelettes de nitrocellulose, portant des protéines séparées par électrophorèse et provenant de particules de HIV purifiées (LAV BLOT, SANOFI DIAGNOSTICS PASTEUR), ont été mises à incuber avec des échantillons de sérum et leur réactivité a été évaluée selon les protocoles recommandés par le fabricant. Les résultats obtenus sont les suivants : Bandelette 1 : protéines spécifiques de VIH-2, qu'on a fait réagir avec un échantillon de sérum de la patiente VIH-1_{(VAU)} obtenu en février 1992. Bandelettes 2-7 : sérums positifs VIH-1 ; sérums de la patiente VIH-1_{(VAU)}: 2: obtenu en novembre 1990; 3 : en décembre 1990; 4 : en février 1991; 5 : en février 1992; 6 : témoin négatif; 7 : témoin positif (sérum d'un individu infecté par le VIH-1). Les noms et la taille des protéines (en kD) sont indiqués dans la marge.

La figure 3 montre un alignement de la séquence d'acides aminés de l'enveloppe de VIH-1_{(VAU)} avec la séquence correspondante de l'isolat de référence VIH-1 LAI (Wain-Hobson, et al. 1985). Les peptides signal, la boucle V3 et l'épitope immunodominant gp41 sont mis en relief par des rectangles hachurés. Le site de clivage entre la glycoprotéine d'enveloppe externe gp120 et la gp41 transmembranaire est indiqué par des flèches. Les lignes verticales entre les lettres d'acides aminés indiquent une identité complète, les deux-points (:) indiquent une forte homologie, et les points (.) indiquent une homologie limitée entre des acides aminés individuels. L'alignement a été effectué en utilisant le programme GAP du progiciel GCG-Wisconsin.

La version originale (1.0) des programmes GAP et BESTFET a été écrite par Paul Haeberli à partir d'une étude détaillée des publications de Needleman et Vunsch (J. Mol. biol. 48, 443-453 (1970) et de Smith et Waterman (Adv. Appl. Math. 2; 482-489 (1981). Les alignements limités ont été mis au point par Paul Haeberli et ajoutés au progiciel pour constituer la version 3.0. Ils ont ensuite été fusionnés en un seul programme par Philip Marquess pour constituer la version 4.0. Les pénalités d'absence d'écart dans l'alignement des protéines ont été modifiées tel que suggéré par Rechid, Vingron et Argos (CABIOS 5 ; 107-113 (1989).

L'alignement de la figure 3 révèle de nombreuses zones de divergence poussée, avec quelques domaines conservés çà et là. Ces régions conservées correspondent grossièrement aux domaines également conservés dans les isolats VIH-1 classiques (Alizon, et al. 1986, Benn, et al. 1985). Parmi les domaines divergents, la boucle V3, appelée également déterminant principal de neutralisation (Javaherian, et al. 1990, Javaherian, et al. 1989, Matsushita, et al. 1988) est clairement l'un des plus divergents, bien que les deux cystéines définissant la boucle soient conservées. La séquence de la coiffe de la boucle, GPGRAF pour le VIH-1 LAI est GPMAWY chez le VIH-1_{(VAU)}. Ce motif de la coiffe est identique à celui de l'isolat du groupe O camerounais HIV_{ANT70} (Van den Heasevelde, et al. 1994), mais est différent de celui de l'autre isolat du groupe O, HIV_{MVP5180} (Gürtler, et al. 1994), pour lequel le motif est GPMRWR.

Dans l'ensemble de l'enveloppe, 29 sites de N-glycosylation potentiels ont été identifiés au total, dont 13 sont conservés comparativement à d'autres protéines d'enveloppe VIH-1. On a également trouvé 19 cystéines conservées au total, ce qui indique que l'architecture globale de repliement de la protéine est conservée, mais on a trouvé 5 cystéines non conservées.

La figure 4 montre l'alignement multiple des peptides immunodominants dans le segment extracellulaire de la glycoprotéine d'enveloppe transmembranaire de différents isolats VIH-1. Toutes les séquences sont comparées à la séquence de référence VIH-1 LAI. Des traits d'union indiquent une identité au VIH-1LAI. L'alignement a été effectué à l'aide du programme PILEUP du progiciel GCG-Wisconsin.

Dans le programme PILEVP, la stratégie de rassemblement représentée par le dendrogramme est nommée UPGMA, qui signifie en anglais « Unweighted pair-group method using arithmetic averages » (Smith, P.H.A Sokal, R.R. (1973) dans Numerical Taxonomy (p.p. 230-234), W.H. Freeman and Company, San Francisco, California, USA). Chaque alignement en pair de PILEVP utilise la méthode de Needleman et Wunsch (Journal of Molecular Biology 48; 443-453 (1970)).

Comme le montre la figure 4, la séquence d'acides aminés de l'épitope immunodominant de la portion extérieure de la protéine TM (Gnann, et al. 1987) est sensiblement différente de celle d'autres isolats VIH-1 et VIH-2. Toutefois, elle a gardé la plupart des acides aminés qui se sont révélés conservés entre les virus VIH-1 et VIH-2.

On a trouvé que certains acides aminés particuliers n'étaient conservés qu'entre les virus du groupe O : tel est le cas de la lysine en position 21 sur un peptide de 26 acides aminés, de la thréonine en position 7 et de l'asparagine en position 11. Ces différences pourraient expliquer l'absence de détection d'antigènes d'enveloppe VIH-1 classiques par un des sérums de la patiente VIH-1_{(VAU)} et également probablement par celui de patients infectés par d'autres virus de groupe O. Globalement, la comparaison entre les séquences d'enveloppe VIH-1 LAI et VIH-1_{(VAU)} a montré une identité de 50 %. La séquence d'enveloppe VIH-1_{(VAU)} a également été comparée à celle d'autres représentants HIV dont les deux membres du groupe O du VIH-1 décrits et séquencés : VIH-1_{ANT70} et VIH-1_{MVP5180}, et SIV. Les résultats de cette analyse, présentés sur le Tableau 1, établissent que le VIH-1_{(VAU)} appartient au groupe O. L'enveloppe VIH-1_{(VAU)} est identique à 70 % à l'enveloppe VIH-1_{ANT70} et identique à 71 % à VIH-1M_{VP5180}. Parmi les sous-types VIH-1 les plus courants, l'identité au niveau de l'enveloppe est comparable, allant de 74 % à 80 %.

| | | | | | | | | | | |
|---|---|---|---|---|---|---|---|---|---|---|
| VIH-1_{EU} | 77 | | | | | | | | | |
| VIH-1_{MAL} | 76 | 80 | | | | | | | | |
| VIH-1_{U455} | 75 | 74 | 76 | | | | | | | |
| SIV_{CPZGAB} | 61 | 62 | 63 | 63 | | | | | | |
| VIH-2_{ROD} | 38 | 39 | 39 | 40 | 39 | | | | | |
| SIV_{MAC251} | 37 | 37 | 38 | 38 | 38 | 74 | | | | |
| SIV_{AGMTYO} | 37 | 36 | 37 | 38 | 40 | 46 | 47 | | | |
| HIV_{ANT70} | 51 | 52 | 52 | 52 | 53 | 33 | 32 | 33 | | |
| HIV_{MVP5180} | 51 | 52 | 54 | 51 | 52 | 36 | 33 | 34 | 70 | |
| HIV_{VAU} | 50 | 51 | 52 | 51 | 54 | 35 | 34 | 35 | 70 | 71 |
| | VIH-1_{LAl} | VIH-1_{EU} | VIH-1_{MAL} | VIH-1_{U455} | SIV_{CPZQAB} | VIH-2_{ROD} | SIV_{MAC251} | SIV_{AGMTYO} | HIV_{ANT70} | HIV_{MVP5180} |

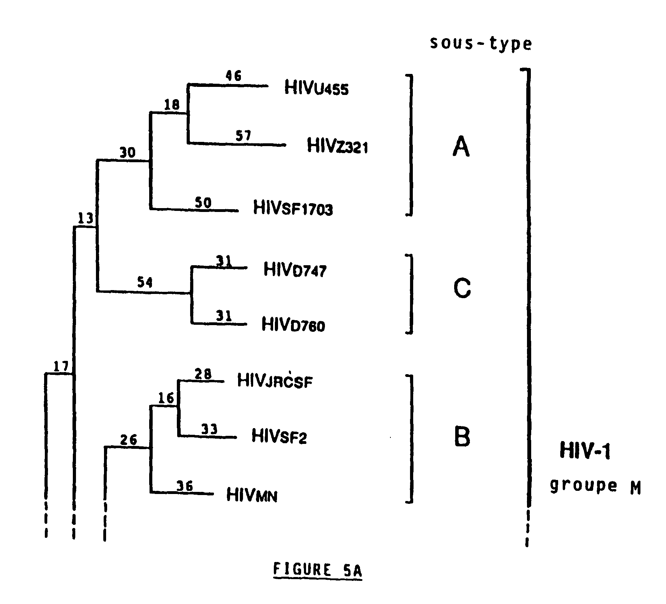

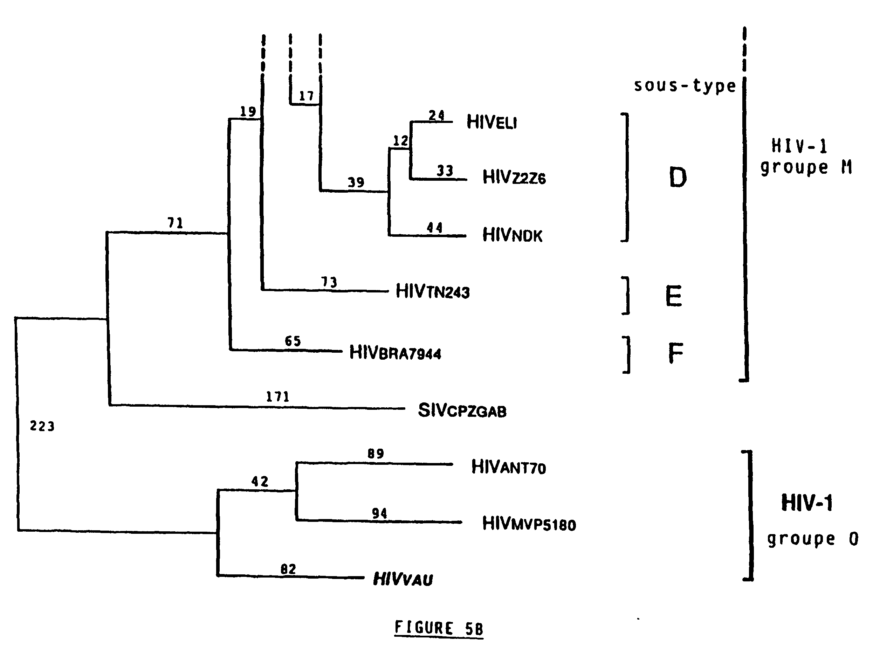

La relation entre le VIH-1_{(VAU)}, d'autres membres de la phylogénie des VIH-1 et les deux virus récemment décrits du groupe O a été analysée par construction d'un arbre phylogénétique de parcimonie non pondérée à l'aide de la séquence nucléotidique de la région transmembranaire *env*. Le résultat de cette analyse est représenté sur la fig. 5 dans laquelle les nombres indiquent le nombre de changements nucléotidiques. La figure 5 montre que le VIH-1_{(VAU)} est à peu près équidistant des deux autres virus du groupe O, et que, globalement, ces trois virus semblent être approximativement équidistants les uns des autres. En effet, le nombre de changements nucléotidiques entre le VIH-1_{MVP5180} et le VIH-1_{(VAU)} est de 218 dans le segment de génome analysé, alors que la distance est de 183 entre le VIH-1_{MVP5180} et le VIH-1_{ANT70}, et de 213 entre le VIH-1_{ANT70} et le VIH-1_{(VAU)}. Ce profil de divergence est très similaire à ce qu'on trouve dans l'ensemble des autres sous-types VIH-1, où le nombre de changements nucléotidiques uniques que l'on trouve entre deux sous-types distincts va de 157 (sous-type E au sous-type F) à 219 (sous-type A au sous-type D).

Le tableau 1 montre la comparaison des séquences d'enveloppe de différents virus apparentés au VIH-1. Les nombres indiquent le pourcentage d'identité en acides aminés entre des séquences d'enveloppe, tels qu'ils ont été calculés à l'aide du programme GAP du progiciel GCG-Wisconsin. *: pour le HIV_{ANT70}, seule la protéine d'enveloppe externe a été utilisée dans la comparaison.

### Compositions comprenant des antigènes VIH-1 _{(VAU)}

De façon générale, l'invention concerne toute composition utilisable en détection in vitro de la présence dans un fluide biologique, notamment de personnes ayant été mises au contact du VIH-1_{(VAU)}, ou d'anticorps contre l'un au moins des antigènes VIH-1_{(VAU)}. Cette composition peut être appliquée au diagnostic sélectif de l'infection par un VIH-1 du groupe O, en mettant en oeuvre des techniques de diagnostic telles qu'elles sont décrites dans les demandes de brevet EP 84401.834 et EP 87400151.4. Dans le contexte de la présente invention, on utilise tout constituant comprenant des déterminants antigéniques capables d'être reconnus par des anticorps produits contre le VIH-1_{(VAU)}, par exemple des antigènes ou peptides recombinants ou peptides synthétisés chimiquement définis à partir de la séquence de l'enveloppe VIH-1_{(VAU)}. A cet égard, l'invention concerne plus particulièrement des compositions contenant l'une au moins des protéines d'enveloppe du virus VIH-1_{(VAU)}. On mentionnera, à titre d'exemples de compositions, celles qui contiennent des protéines, glycoprotéines ou peptides de la protéine d'enveloppe correspondant à l'ensemble de la région 590-620 de la protéine gp41 du VIH-1_{(VAU)} ou aux parties de cette région spécifiques du VIH-1_{(VAU)} tels que les peptides -TFIQN- ou -WGCKNR-.

L'invention concerne également des compositions associant des protéines et/ou glycoprotéines et/ou peptides VIH-1_{(VAU),} recombinants ou synthétiques, avec des protéines et/ou glycoprotéines et/ou peptides VIH-1 et/ou VIH-2 et/ou d'un autre VIH-1 groupe O obtenus par extraction ou dans des lysats ou par recombinaison ou par synthèse chimique et/ou de peptides dérivés de ces protéines ou glycoprotéines et susceptibles d'être reconnus par des anticorps induits par le virus VIH-1 et/ou VIH-2 et/ou VIH-1, groupe O.

Les compositions diagnostiques contenant des déterminants antigéniques capables d'être reconnus par des anticorps dirigés contre le VIH-1_{(VAU)}, en particulier les compositions peptidiques, peuvent être incluses dans ou associées à des compositions ou des trousses déjà disponibles pour la détection de l'infection par des rétrovirus VIH-1 et/ou VIH-2, de façon à étendre le spectre de détection des trousses à la détection des rétrovirus VIH-1 du groupe O.

A titre d'exemples non limitatifs :
- soit des protéines du noyau (core), particulièrement les protéines gag, pol, VIH-1 et VIH-2 ou des peptides de celles-ci, et des protéines ou peptides d'enveloppe VIH-1_{(VAU)},
- soit des glycoprotéines d'enveloppe VIH-1, des glycoprotéines d'enveloppe VIH-2 et des glycoprotéines d'enveloppe VIH-1_{(VAU)},
- soit des mélanges de protéines et/ou glycoprotéines VIH-1, de protéines et/ou glycoprotéines VIH-2 et de protéines et/ou glycoprotéines d'enveloppe VIH-1_{(VAU)}.

Il est important de noter que, bien que les anticorps de patients infectés par des virus VIH-1 groupe O réagissent fortement avec des antigènes gag et pol de virus VIH-1 groupe M, leur réactivité est pratiquement nulle avec des antigènes d'enveloppe de virus du groupe M. II est donc important que la composition de la présente invention comprenne au moins une protéine ou un peptide d'enveloppe VIH-1_{(VAU)} de façon à ce que ce virus puisse être détecté avec certitude.

De telles compositions, utilisées en diagnostic, aident par conséquent au diagnostic du sida ou des symptômes qui lui sont associés, qui s'étendent sur un plus large spectre des agents étiologiques responsables. Il va sans dire que l'utilisation de compositions de diagnostic qui ne contiennent que des protéines et/ou glycoprotéines d'enveloppe VIH-1_{(VAU)} n'en reste pas moins utile pour la détection plus sélective de la catégorie de rétrovirus qui peut être tenue pour responsable de la maladie.

### Procédés et trousses de diagnostic d'infections causées notamment par le virus VIH-1 _{(VAU)}

La présente invention concerne un procédé de diagnostic in vitro de l'infection par les HIV, agents étiologiques du SIDA et de syndromes apparentés, qui comprend la mise en contact d'un sérum ou d'un autre milieu biologique, provenant d'un patient ou sujet faisant l'objet du diagnostic, avec une composition contenant au moins une protéine, une glycoprotéine ou un peptide VIH-1_{(VAU)} selon les revendications, et la détection d'une réaction immunologique éventuelle. Des exemples de telles compositions ont été décrits plus haut.

Des méthodes préférées mettent en jeu, par exemple, des réactions immunoenzymatiques de type ELISA ou d'immunofluorescence. Les détections peuvent se faire par immunofluoresence directe ou indirecte ou des dosages immunoenzymatiques directs ou indirects.

De telles détections comprennent par exemple :
- le dépôt de quantités déterminées de l'extrait ou des compositions antigéniques visées conformes à la présente invention dans les puits d'une microplaque;
- l'introduction dans chaque puits d'un sérum dilué ou non, susceptible de contenir les anticorps dont la présence doit être détectée in vitro ;
- l'incubation de la microplaque ;
- le lavage soigneux de la microplaque avec un tampon approprié ;
- l'introduction dans les puits de la microplaque d'anticorps marqués spécifiques anti-immunoglobuline humaine, le marquage étant réalisé par une enzyme choisie parmi celles qui sont capables d'hydrolyser un substrat de telle sorte que ce dernier subit alors une modification de son absorption des radiations, au moins dans une bande de longueur d'onde déterminée, et
- la détection, de préférence de façon comparative par rapport à un témoin, de l'importance de l'hydrolyse du substrat en tant que mesure des risques potentiels ou de la présence effective de l'infection.

La présente invention est également relative à des nécessaires ou trousses de diagnostic de l'infection par le virus VIH-1_{(VAU)}, lesquels comprennent notamment :
- un extrait, une fraction plus purifiée, ou un antigène synthétique dérivé des types de virus indiqués ci-dessus, cet extrait fraction ou antigène marqué, par exemple, de façon radioactive, enzymatique, fluorescente ou autre,
- des anticorps antiimmunoglobulines humaines ou une protéine A (fixés de façon avantageuse sur un support insoluble dans l'eau) telles que des billes par exemple d'agarose) ou des puits de microplaque, etc.),
- éventuellement un échantillon de fluide biologique ou cellules obtenu à partir d'un sujet témoin négatif :

- des tampons et, le cas échéant, des substrats pour la visualisation du marquage.

L'invention a aussi pour objet des compositions immunogènes capables d'induire la formation d'anticorps reconnaissant des antigènes pouvant être obtenus par synthèse chimique ou par recombinaison.

### Sérologie

La capacité des anticorps sériques de la patiente infectée par le VIH-1_{(VAU)} à réagir avec des préparations antigéniques VIH-1 a été évalué à l'aide de diverses trousses disponibles dans le commerce : SANOFI DIAGNOSTICS PASTEUR, (GENELAVIA MIXT) ABBOTT, WELLCOME, et BEHRING. La réactivité de ces anticorps avec différentes protéines VIH-1 a été examinée à l'aide de la trousse de Western-blot de SANOFI DIAGNOSTICS PASTEUR, suivant les protocoles recommandés par le fabricant.

Plus précisément, le sérum de la patiente a été examiné plusieurs fois à l'aide de trousses ELISA spécifiques VIH-1. Il a d'abord été testé et s'est avéré être positif en 1990, étant noté 7,33 (ce chiffre correspond au rapport de la DO mesurée sur la DO du bruit de fond) avec la trousse de SANOFI DIAGNOSTICS PASTEUR, 3,50 avec la trousse ABBOTT et 2,70 avec la trousse WELLCOME. Lors de l'utilisation des réactifs spécifiques à la fois du VIH-1 et du VIH-2, le sérum a été noté 1,42 avec la trousse Behring et 4,40 avec la trousse WELLCOME.

La capacité du sérum de la patiente à réagir à différentes dates avec différentes protéines de structure VIH-1 a été étudié à l'aide du dosage par immunoempreinte LAV BLOT VIH-1, test commercialisé par SANOFI DIAGNOSTICS PASTEUR. Comme le montre la figure 5 avec tous les échantillons de sérum testés, on n'a remarqué qu'une très faible réactivité du sérum avec les protéines *env* VIH-1 gp160 et gp120. Toutefois, le sérum a réagi fortement avec les protéines gag du VIH-1 p55 (précurseur de gag) et p24 (CA), et avec les produits pol p66 (RT) et p34 (IN). En immunoempreinte VIH-2, on n'a détecté qu'une très faible réactivité avec la p26 gag.

Ceci illustre que la détection d'anticorps spécifiques du groupe O avec des trousses de diagnostic sérique disponibles dans le commerce doit être soigneusement contrôlée. Bien que des anticorps sériques de patients infectés par des virus du groupe O présentent de fortes réactions croisées avec les antigènes gag et pol du groupe M, ils présentent peu ou pas de réactions avec les antigènes d'enveloppe de groupe M. On peut, par conséquent, supposer qu'une proportion significative de ces patients pourrait ne pas être détectée lors de l'utilisation de certaines trousses à base de réactifs antigéniques d'enveloppe du groupe M. En effet, dans une étude préliminaire récente de plusieurs sérums de patients infectés par le groupe O, il a été trouvé que la capacité de détecter des anticorps spécifiques du groupe O était très différente selon la trousse de détection utilisée (Loussert-Ajaka, I., Ly, T.D., Chaix, M.L., Ingrand, D., Saragosti, S., Courroucé, AM., Brun-Vézinet, F. and Simon, F. (1994). VIH-1/VIH-2 seronegativity in VIH-1 subtype O infected patients. Lancet. 343, 1393-1394.). Ceci implique qu'une étude soignée et poussée de la réactivité d'un grand nombre de sérums du groupe O avec toutes les trousses de diagnostic disponibles sur la marché est nécessaire.

### Compositions comprenant des anticorps polyclonaux ou monoclonaux préparés à partir des antigènes recombinants ou synthétiques du virus VIH-1 _{(VAU)}.

L'invention concerne un sérum susceptible d'être produit chez l'animal par inoculation à celui-ci du VIH-1_{(VAU)}, particulièrement les épitopes antigéniques du VIH-1_{(VAU)} et plus particulièrement les épitopes antigéniques de la protéine d'enveloppe du virus VIH-1_{(VAU)}. L'invention concerne plus particulièrement les anticorps polyclonaux plus spécifiquement orientés contre chacun des antigènes, notamment protéines ou glycoprotéines selon les revendications. Elle concerne aussi des anticorps monoclonaux produits par différentes techniques, ces anticorps monoclonaux étant orientés respectivement plus spécifiquement contre les protéines d'enveloppe du VIH-1_{(VAU)}.

Ces anticorps polyclonaux ou monoclonaux sont utilisables dans différentes applications. On mentionnera essentiellement leur utilisation pour neutraliser les protéines correspondantes, voire inhiber l'infectivité du virus entier. Ils peuvent également être utilisés par exemple pour mettre en évidence les antigènes viraux dans les préparations biologiques ou pour réaliser des opérations de purification des protéines et/ou glycoprotéines correspondantes, par exemple lors de l'utilisation dans des colonnes de chromatographie d'affinité.

A titre d'exemple, des anticorps anti-enveloppe sont des réactifs utilisables en diagnostic, en particulier pour la détection de particules VIH-1 groupe O par ELISA de capture d'antigène.

L'invention concerne des anticorps dirigés contre un ou des antigènes du virus VIH-1_{(VAU)} produits à partir de séquences d'acides aminés du VIH-1_{(VAU)} selon les revendications. Des techniques d'obtention d'anticorps à partir d'épitopes antigéniques semblables aux épitopes antigéniques du virus VIH-1(_{VAU}) de la présente invention ont été décrites précédemment.

La technique de préparation d'anticorps décrite dans la publication de ULMER et al., 1993 peut être utilisée par l'homme du métier pour préparer les anticorps de la présente invention, les modifications permettant d'adapter cette technique aux antigènes de la présente invention faisant partie des connaissances de l'homme du métier.

### Etude de l'immunoréactivité du peptide vau

L'immunoréactivité du peptide vau a été vérifiée, après avoir préparé des plaques ELISA expérimentales, selon un protocole établi pour un test de screening des anticorps anti-HIV. Ce test repose sur la détection d'une phase solide préparée avec le peptide mimant l'épitope immunodominant de la glycoprotéine d'enveloppe du virus VIH-1 groupe (ou sous-groupe) O, isolat VAU. La mise en oeuvre du test a été calquée sur le protocole proposé par la trousse GENELAVIA® MIXT, avec les réactifs de cette trousse.

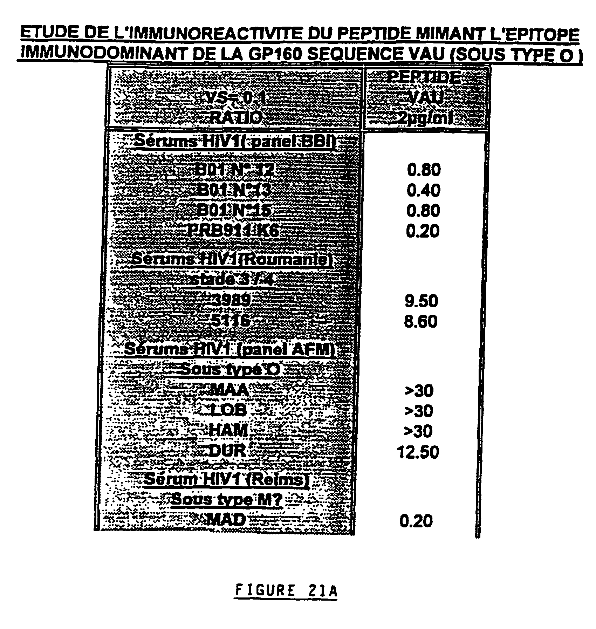

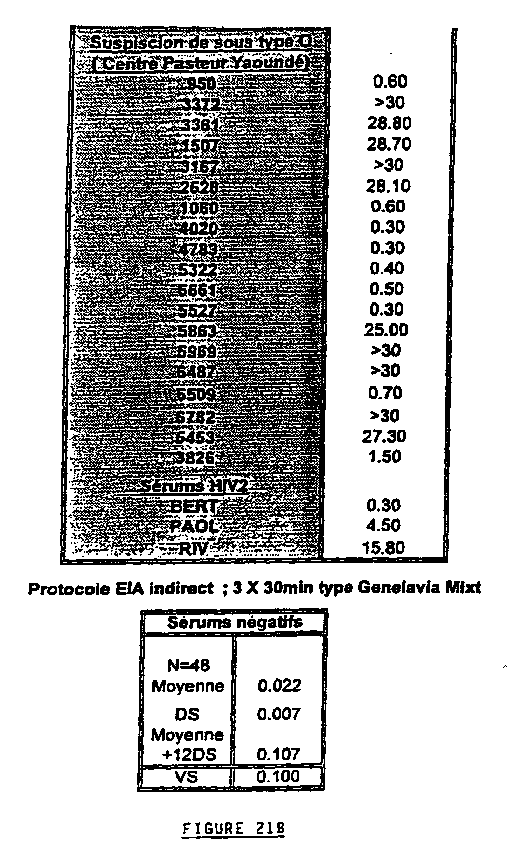

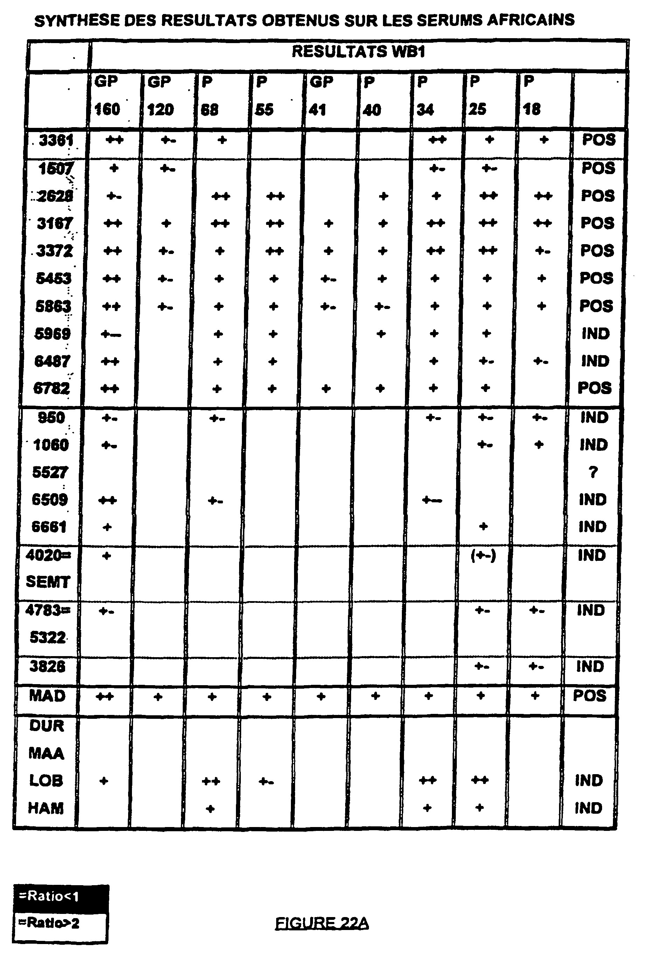

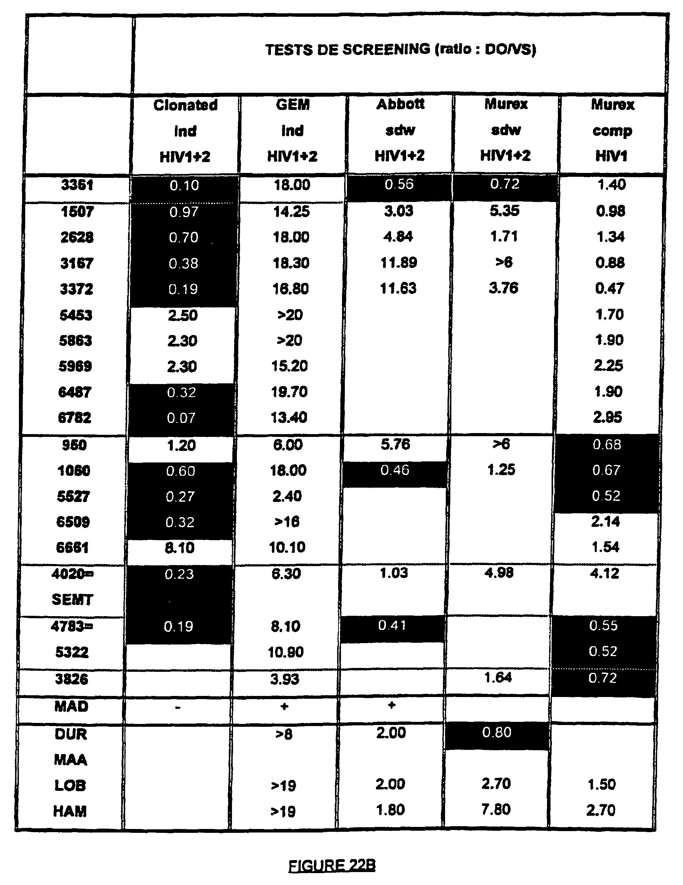

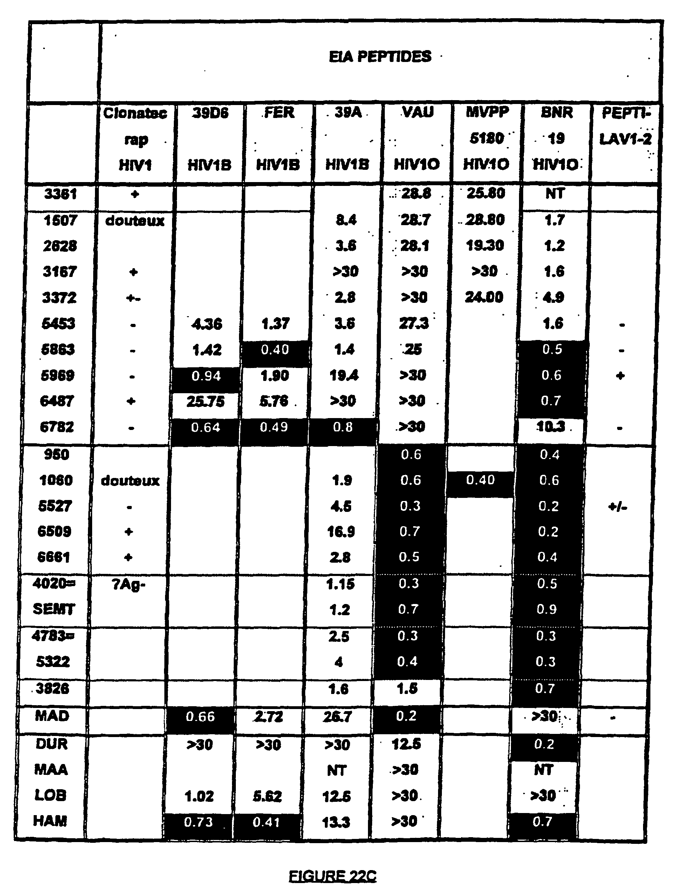

Les données expérimentales rassemblées dans les deux tableaux des figures 21 et 22 démontrent que :
a) les quatre sérums prélevés chez des patients contaminés par le virus VIH-1 groupe (ou sous-groupe) O sont très réactifs sur le peptide vau ;
b) les dix sérums censés prélevés chez des patients contaminés par le virus VIH-1 (groupe ou sous-groupe) 0, parmi les 19 sérums transmis par l'institut Pasteur de Yacoundé sont également fortement réactifs sur ce même peptide :
c) les sérums (4 échantillons) prélevés chez des sujets contaminés par le virus VIH-1 sous-type B (en phase aiguë) ne sont pas réactifs sur le peptide vau ;
d) les sérums prélevés chez des donneurs de sang asymptomatiques (48 échantillons testés) ne sont pas réactifs sur le peptide vau ; Ces données expérimentales, bien que limitées (vu la paupicité des échantillons positifs en anticorps VIH-1 groupe (ou sous-groupe O) attestent de la sensibilité et de la spécificité du peptide sélectionné.

De ce qui précède, il résulte que la demande décrit aussi la détection du virus VIH-1_{(VAU)} ou d'un variant grâce à la mise en oeuvre des anticorps décrits précédemment dans un procédé faisant appel à différentes étapes, ces étapes étant spécifiquement prévues pour faire apparaître les propriétés caractéristiques du virus VIH-1_{(VAU)}.

L'invention concerne aussi la détection du virus VIH-1_{(VAU)} par hybridation moléculaire.

D'une façon générale, ce procédé de détection du virus VIH-1_{(VAU)} ou d'un variant dans des échantillons de sérum ou d'autres liquides biologiques ou tissus, obtenus à partir de patients susceptibles d'être porteurs du virus VIH-1_{(VAU)} comprend les étape suivantes:
- la fabrication d'au moins une sonde éventuellement marquée ;
- au moins une étape d'hybridation conduite dans des conditions permettant l'hybridation par mise en contact de l'acide nucléique de l'échantillon du patient suspect avec ladite sonde marquée et éventuelle immobilisation du complexe formé sur un support solide approprié,
- le cas échéant lavage dudit support solide avec une solution de lavage adéquate,
- la détection dudit complexe et donc de la présence ou non du virus VIH-1_{(VAU)} par méthode appropriée de détection connue de l'homme du métier.

Dans un autre mode de réalisation préféré du procédé selon l'invention, l'hybridation précitée est conduite dans des conditions non stringentes et le lavage du support (membrane) est réalisé dans des conditions adaptées à celles de l'hybridation.

En utilisant une sérologie ou une technique d'amplification génique telle que la Polymerase Chain Réaction (PCR) spécifique, l'importance de l'épidémie VIH-1 du groupe O a été évaluée précisément. Il a été trouvé que 5 à 10 % de patients infectés par un VIH-1 au Cameroun sont en réalité infectés par des virus du groupe O. Toutefois, à part l'isotat de virus décrit ici, la propagation de virus du groupe O en dehors de l'Afrique de l'Ouest Centrale n'a pas été documentée. La patiente chez qui le VIH-1_{(VAU)} a été isolé a toujours vécu en France et n'a jamais voyagé en Afrique. Jusqu'ici, nous n'avons aucune preuve précise concernant l'origine de son infection, mais ce cas indique qu'une certaine propagation des virus du groupe O en Europe s'est déjà produite.

L'échantillon biologique peut être constitué notamment de sang, de plasma, de sérum ou de tout autre extrait biologique. Les compositions ci-dessus sont utilisables pour la détection d'anticorps dans un des échantillons biologiques susdits.

La demande divulgue également une méthode de diagnostic in vitro d'une infection due spécifiquement à un rétrovirus du type HIV, caractérisée par les étapes de:
- mise en contact d'un échantillon biologique susceptible de contenir des anticorps produits à la suite d'une infection par un rétrovirus VIH-1 groupe (ou sous-groupe) O, avec un peptide tel que précédemment défini, ou avec une composition peptidique telle que précédemment définie, dans des conditions appropriées permettant la formation d'un complexe immunologique du type antigène/anticorps,
- détection de l'éventuelle présence du complexe.

Est décrite par ailleurs, une composition immunogène, caractérisée en ce qu'elle comprend au moins un peptide en association avec un véhicule pharmaceutique acceptable pour la constitution de vaccins.

L'invention se rapporte également à un procédé de préparation des protéines de capside et des glycoprotéines gp41 et gp120 d'une souche rétrovirale selon l'invention, le procédé étant caractérisé par tes étapes suivantes :
- lyse des cellules infectées par un rétrovirus VIH-1 selon l'invention et séparation du surnageant et des cellules infectées ou lyse des culots viraux préparés par centrifugation,
- dépôt de l'extrait cellulaire et/ou de l'extrait viral sur immuno-adsorbant contenant des anticorps purifiés, obtenus à partir d'un sérum de sujet infecté par un rétrovirus selon l'invention, et fixés avantageusement sur un support adapté, ledit sérum de sujet infecté ayant la capacité de réagir fortement avec des protéines d'enveloppe du virus selon l'invention,
- incubation en présence d'un tampon et pendant un temps suffisamment long pour obtenir la formation d'un complexe immunologique antigène/anticorps.
- lavage de l'immuno-adsorbant avec un tampon pour éliminer les molécules non retenues sur le support,
- récupération des protéines antigéniques recherchées.

Selon un premier mode de réalisation de ce procédé de préparation, la séparation et la récupération des protéines de capside et des glycoprotéines gp41 et gp120 de VIH-1 DUR sont faites par électrophorèse et par électroréduction des protéines.

Selon un autre mode de réalisation de ce procédé de préparation, la récupération des protéines est obtenue par :
- élution des protéines fixées sur l'immuno-adsorbant ci-dessus,
- purification des produits ainsi élués sur une colonne de chromatographie contenant, fixés sur le support de séparation, des anticorps reconnaissant les protéines de capside et les glycoprotéines gp41 et gp120 de VIH-1 groupe (ou sous-groupe) O DUR.

Entre également dans le cadre de l'invention, un procédé de production d'un polypeptide ou d'un peptide selon l'invention, ce polypeptide ou peptide étant obtenu
- soit par l'expression d'un acide nucléique de l'invention,
- soit par synthèse chimique, par addition d'acides aminés jusqu'à l'obtention de ce polypeptide ou de ce peptide.

Les principes et les procédés classiques du génie génétique peuvent être ici utilisés ( « Molecular cloning », Sambrook, Fritsch, Maniatis, CSH 1989)

La demande décrit, un procédé de production d'un acide nucléique précédemment défini, pouvant être produit soit par isolement à partir d'un virus de l'invention, soit par synthèse chimique, soit en utilisant des techniques d'amplification in vitro des acides nucléiques à partir d'amorces spécifiques.

### Détermination d'oligonucléotides spécifiques du groupe O.

A l'aide de la séquence VAU et de sa corrélation aux séquences MVP5180 et ANT70, on a défini des amorces oligonucléotidiques s'efforçant d'être spécifiques du sous-groupe O dans son entièreté pour la région V3 et la région gp41. Celles-ci ont permis d'amplifier la souche DUR et ont en conséquence constitué une solution au problème d'amplification rencontré. La position ainsi que la séquence de ces amorces HIV sous-groupe O sont représentées à la figure 13. Ces amorces permettent l'obtention d'une bande d'amplification visible en coloration au bromure d'éthidium avec une seule étape de 30 cycles de PCR. Des séquences partielles ont été obtenues:
- GAG : 513 paires de bases (171 acides aminés) = Seq ID N° 9
- gp 120 boucle V3 : 525 paires de bases (175 acides aminés) = Seq ID N°10
- gp41 région immunodominante : 312 paires de bases (104 acides aminés) = Seq ID N° 11.

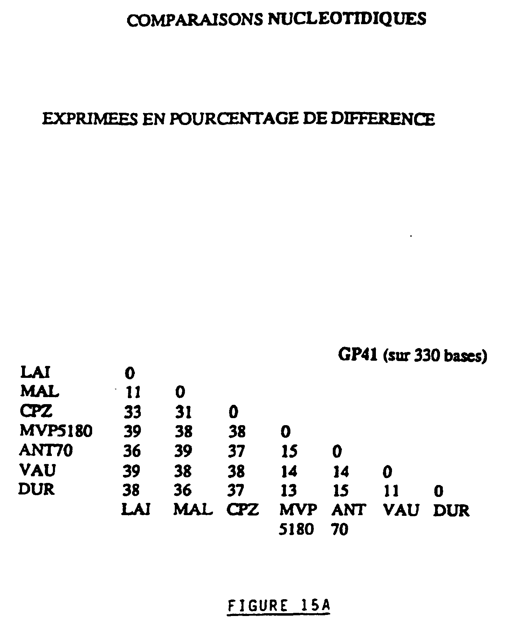

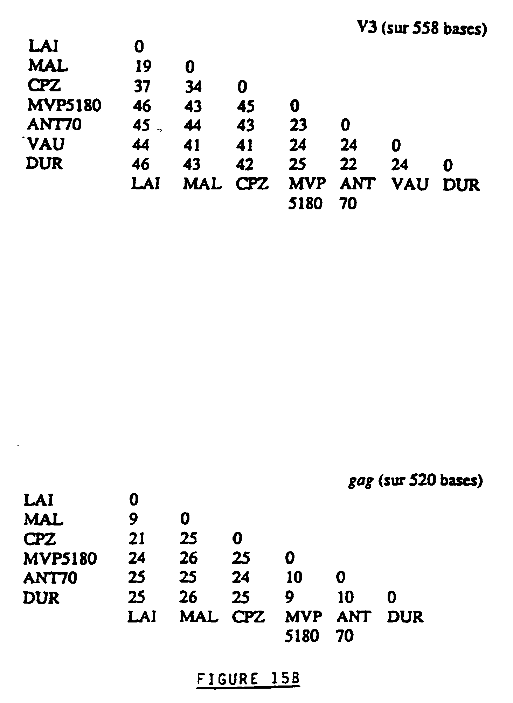

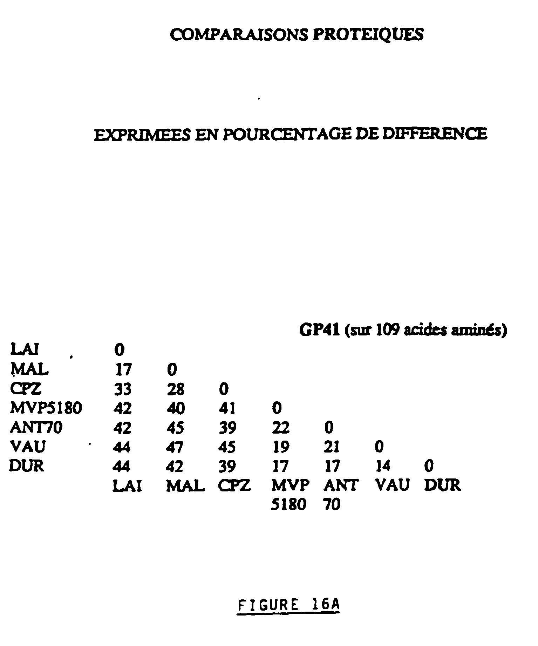

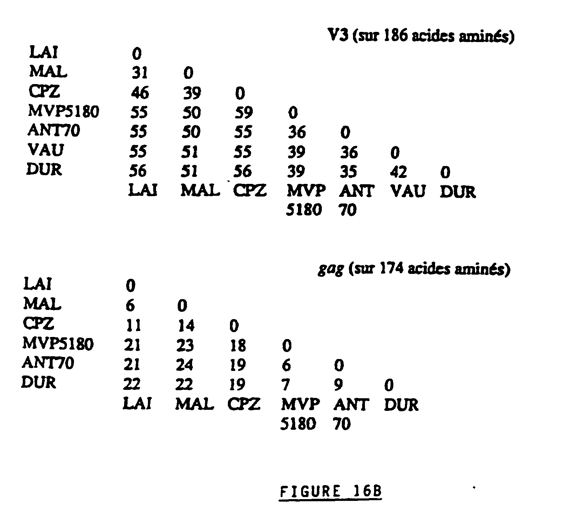

Les comparaisons nucléotidiques (figure 15) et protidiques (figure 16) des séquences DUR aux séquences MVP5180, ANT et VAU pour le sous-groupe O, LAI pour le consensus VIH-1, MAL séquence africaine VIH-1 représentative, et CPZ pour le CIV du chimpanzé gabonais, montrent que DUR est aussi éloigné des autres VIH-1 groupe (ou sous-groupe) O publiés que ceux-ci le sont entre eux.

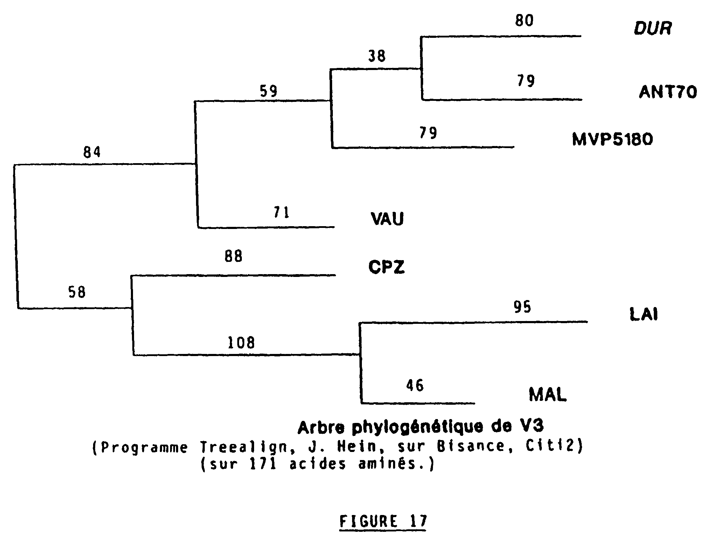

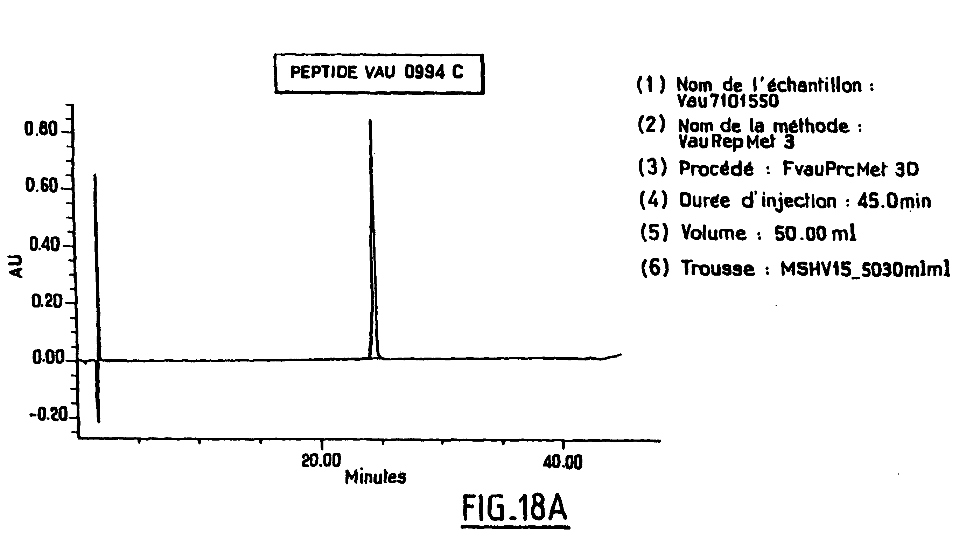

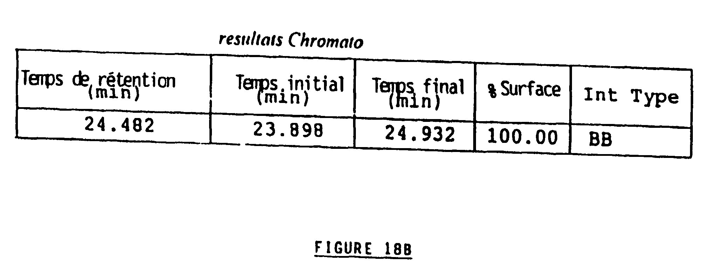

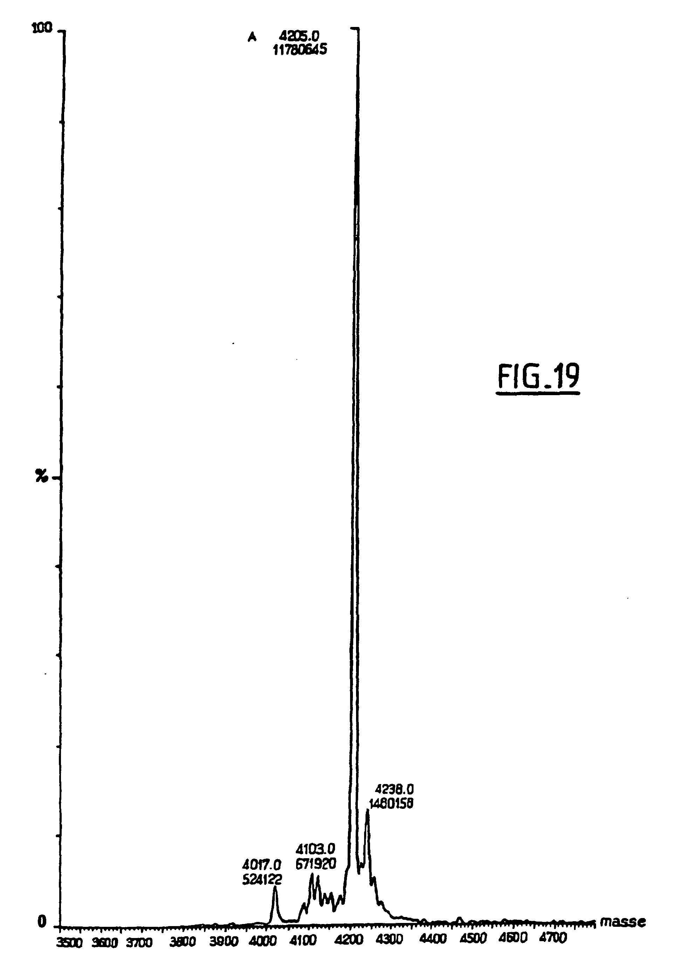

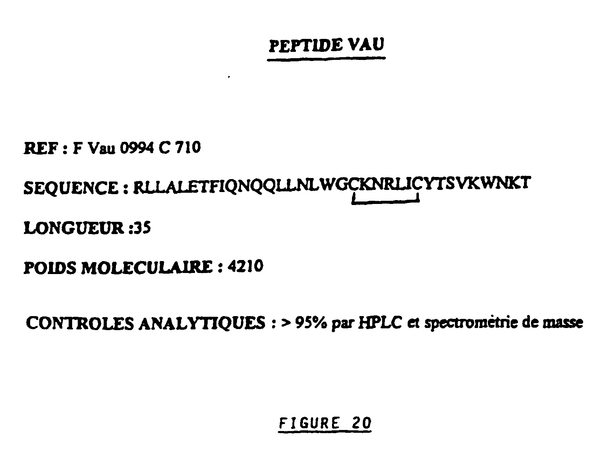

Les différences sont moindres dans la région GAG et maximales dans la région de la boucle V3 de gp120, où la comparaison protéique atteint 40% de différence (figure 16). Les arbres phylogéniques confirment d'une part que la souche DUR fait partie du sous-groupe O, et d'autre part l'importance des différences entre les diverses souches O décrites, sans pour autant que des embranchements de sous-types ne se dégagent nettement (figure 17) .

### Comparaison des séquences GAG :

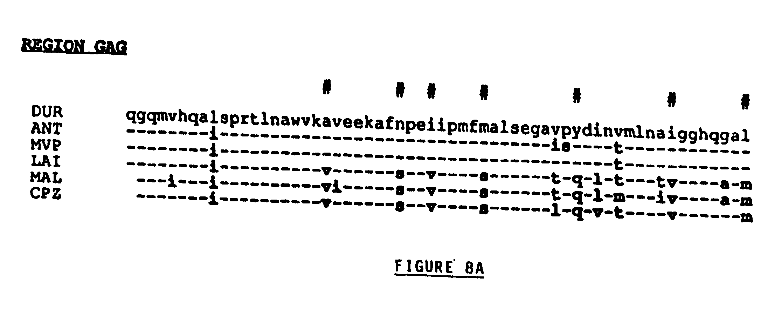

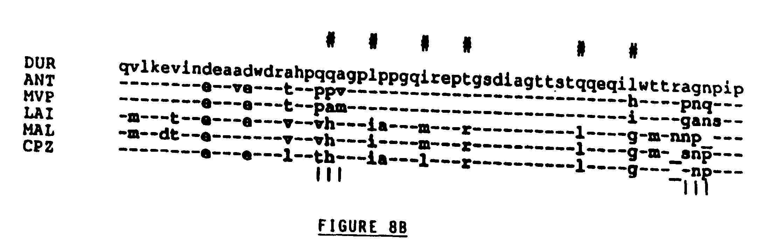

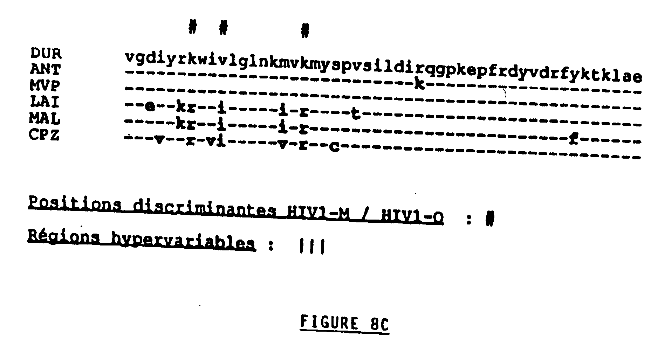

Lors de la comparaison de la séquence GAG obtenue aux deux autres souches O publiées ANT70 et MVP5180 ainsi qu'aux séquences représentatives du groupe M (figure 8), on a pu constater qu'il existe un consensus O sur plusieurs zones, distinct du consensus M dans les mêmes zones. On peut aussi relever deux zones hyper-variables, plus variables pour O que pour M, et quelques différences ponctuelles pour l'une ou l'autre souche. Néanmoins, les régions SPRT....SEGA, MLNAI...KEVIN, GPLPP....CQEQI, VGD....SPV paraissent discriminatives du consensus O versus le consensus M.

Les régions QQA et LWTTRAGNP sont des régions hyper-variables. La souche VIH-1 groupe (ou sous-groupe) O DUR se singularise en trois positions vis-à-vis du consensus M et O (L pour 1 et 2 fois pour E) et prend un acide aminé spécifique en trois positions hyper-variables isolées V position L9 ; A position A77 ; L position 110.

En outre, il est possible de définir dans la région GAG des segments communs au groupe O et au groupe M tels que SPRTLNAWVK, GSDIAGTTST et QGPKEPFRDYVDRF.

### Comparaison des séquences de la boucle V3

Cette étude comparative a révélé des différences considérables atteignant 56% de différences protéiques avec le consensus VIH-1 sous-groupe M, et 35 à 42% avec les autres VIH-1 groupe (ou sous-groupe) O.

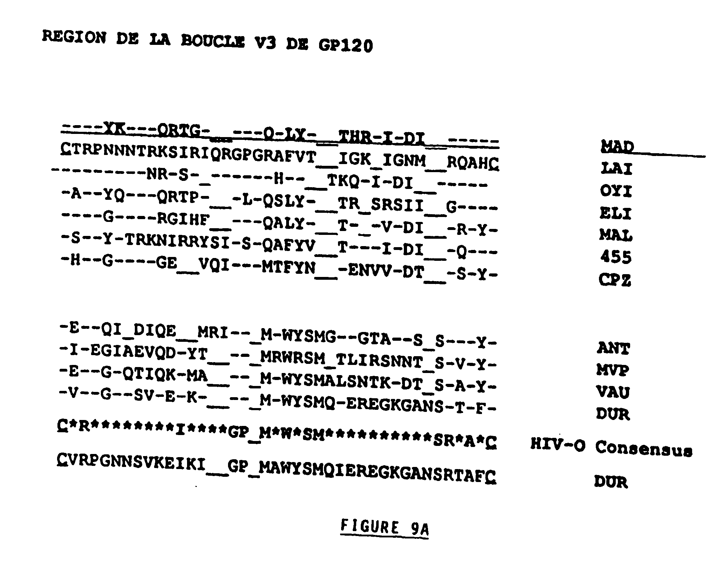

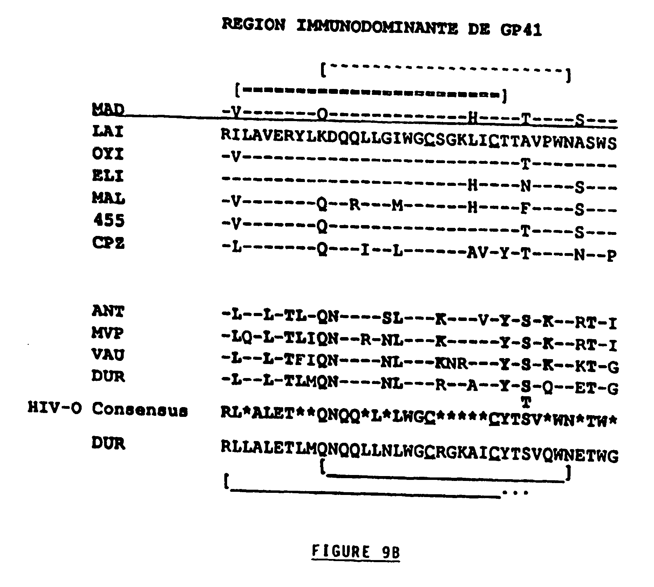

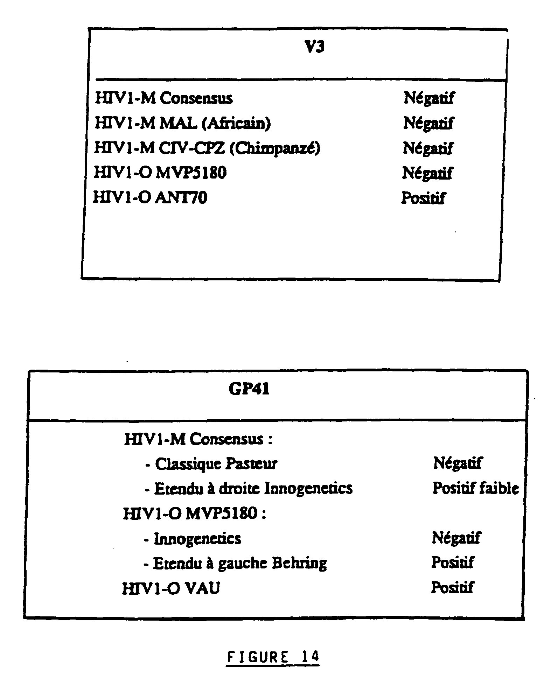

Les alignements de séquences peptidiques des régions de la boucle V3 de gp120 et de la région immunodominante de gp41 est donné à la figure 9. La séquence de l'intérieur de la boucle V3 de la souche DUR diffère substantiellement de celle du consensus VIH-1 sous-groupe M. Elle partage le motif GPMAWYSM avec les souches VAU et ANT70 mais pas avec la souche MVP qui présente deux substitutions : R pour A et R pour Y.

Les parties gauche et droite du reste de la boucle V3 sont nettement différentes de tous les autres HIV connus et ne laissent pas supposer d'autres réactivités croisées. De plus, la boucle V3 de DUR est plus longue d'un acide aminé que les autres séquences O, elles-mêmes plus longues d'un autre acide aminé que les séquences de groupe VIH-1 M.

### Comparaison des alignements concernant la région immunodominante de la gp41

La « mini boucle » de la souche DUR, de séquence CRGKAIC, s'est avérée être très spécifique de cette souche : elle constituerait une épitope (voir figure 9). En outre, cette séquence serait susceptible d'intervenir dans la modification des conditions de dépliage de la glycoprotéine gp41, et en conséquence dans l'infectuosité de la souche.

Une longue séquence de 11 acides aminés flanquant à gauche cette boucle est identique à la séquence VAU. On peut noter un polymorphisme de la souche DUR pour une position S ou T selon les clones analysés.

Sont également représentés dans la figure 9 des peptides correspondants issus d'autres souches rétrovirales connues.

La souche DUR permet également de définir un consensus HIV sous-groupe O de la région gp41 dont plusieurs régions homologues suffisamment longues pourraient être utilisées. Ces régions homologues sont entre autres: RL*ALET,QNQQ, LWGC, CYTV (* représentant un acide aminé variable).

### Corrélations sérologiques:

L'antisérum anti-DUR ne réagit pas avec les peptides de la boucle V3 du consensus VIH-1-M, du VIH-1 MAL, du VIH-1 CPZ ou du VIH-1 groupe (ou sous-groupe) 0 MVP5180, mais réagit cependant avec le peptide de la boucle V3 de l'VIH-1-O ANT70. Concernant la région immunodominante gp41, il ne réagit pas avec le consensus VIH-1 sous-groupe M «classique », mais réagit cependant, faiblement mais de façon surprenante, avec le consensus VIH-1 sous-groupe M étendu à droite.

### REFERENCES

Agut, H., Candotti, D., Rabanel, B., Huraux, J., Remy. G., Ingrand, D., Tabary, T., Chippaux, C., Chamaret, S., Guétard, D., Dauguet, C. et Montagnier, L. (1992). Isolation of atypical VIH-1-related retrovirus from AIDS patient (L'isolement de rétrovirus atypique apparenté au VIH-1 de patient atteint du SIDA). Lancet. 340, 681-682.
Alizon, M., Wain-Hobson, S., Montagnier, L. et Sonigo, P. (1986). Genetic variability of the AIDS virus : nucleotide sequence analysis of two isolates from African patients (Variabilité génétique du virus du SIDA: analyse de la séquence nucléotidique de deux isolats de patients africains). Cell. 46, 63-74.
Barré-Sinoussi, F., Chermann, J.C., Rey, F., Nugeyre, M. T., Chamaret, S., Gruest, J., Dauguet, C., Axler-Blin, C., Brun-Vezinet, F., Rouzioux, C., Rozenbaum, W. et Montagnier, L. (1983). Isolation of a T-lymphotropic retrovirus from a patient at risk for acquired immune deficiency syndrome (AIDS) (Isolement d'un rétrovirus lymphotrope T d'un patient présentant un risque de symptôme de l'immunodéficience acquise (SIDA)). Science. 220, 868-871.
Benn, S., Rutledge, R., Folks, T. et al, e. (1985). Genomic heterogeneity from AIDS retroviral isolates from North America and Zaire (Hétérogénéité génomique d'isolats rétroviraux de SIDA de l'Amérique du Nord et du Zaïre). Science. 230, 949-951.
Clavel, F. et Chameau, P. (1994). Fusion from without directed by Human immunodeficiency virus particles (Fusion de l'extérieur dirigée par des particules du virus de l'immunodéficience humaine). J. Virol. 68, 1179-1185.
Clavel, F., Guétard, D., Brun-Vézinet, F., Chamaret, S., Rey, M. A., Santos-Ferreira, M. O., Laurent, A. G., Dauguet, C., Katlama, C., Rouzioux, C., Klatzmann, D., Champalimaud, J. L. et Montagnier, L. (1986). Isolation of a new human retrovirus from West African patients with AIDS (Isolement d'un nouveau rétrovirus humain de patients ouest-africains atteints du SIDA). Science. 233, 343-346.
De Cock, K. M., Adjorlolo, G., Epkini, E., Sibailly, T., Kouadio, J., Maran, M., Brattegaard, K, Vetter, K, Doorly, R. et Gayle, H. (1993). Epidemiology and transmission of VIH-2 : why there is no VIH-2 epidemic (Epidémiologie et transmission de VIH-2 : pourquoi il n'y a aucune épidémie de VIH-2). JAMA. 270, 2083-2086.
De Leys, R., Vanderborght, B., Vanden Haesevelde, M., Heyndrickx, L., van Geel, A., Wauters, C., Bernaerts, R., Saman, E., Nijs, P. et Willems, B. (1990). Isolation and partial characterization of an unusual human immunodeficiency retrovirus from two persons of west-central African origin (Isolement et caractérisation partielle d'un rétrovirus inhabituel de l'immunodéficience humaine de deux personnes originaires de l'Afrique de l'Ouest Centrale). J Virol. 64, 1207-1216.
Gnann, J., Cormick, J., Michell, S., Nelson, J. et Oldstone, M. (1987). Synthetic peptide immunoassay distinguishes HIV type 1 and type 2 infections (L'immunodosage par peptide de synthèse distingue les infections de HIV du type 1 et du type 2. Science. 237, 1346-1349.
Grez, M., Dietrich, U., Balfe, P., von Briesen, H., Maniar, J., Mahambre, G., Delwart, E., Mullins, J. et Rubsamen-Waigmann, H. (1994). Genetic analysis of Human Immunodeficiency virus type 1 and 2 (VIH-1 and VIH-2) mixed infections in India reveals a recent spread of VIH-1 and VIH-2 from a single ancestor for each of these viruses (L'analyse génétique d'infections mixtes du virus de l'immunodéficience humaine du type 1 et 2 (VIH-1 et VIH-2) en Inde révèle une propagation récente de VIH-1 et de VIH-2 à partir d'un seul ancêtre pour chacun de ces virus). J. Virol. 68, 2161-2168.
Gürtler, L. G., Hauser, P. H., Eberle, J., von Brunn, A., Knapp, S., Zekeng, L., Tsague, J. M. et Kaptue, L. (1994). A new subtype of Human Immunodeficiency Virus type 1 (MVP-5180) from Cameroon (Un nouveau sous-type de virus de l'immunodéficience humaine du type 1 (MVP-5180) du Cameroun). J Virol. 68, 1581-1585.
Harada, S., Koyanagi, Y. et Yamamoto, N. (1985). Infection of HTLV-III/LAV in HTLV-I-carrying cells MT-2 and MT-4 and application in a plaque assay (Infection de HTLV-III/LAV dans des cellules MT-2 et MT-4 porteuses de HTLV-I et application dans un dosage sur plage). Science. 229, 563-566.
Hirt, B. (1967). Selective extraction of polyoma DNA from infected mouse cell cultures (Extraction sélective d'ADN de polyome à partir de cultures de cellules de souris infectées). J. Mol. Biol. 26, 365-369.
Huct, T., Cheynier, R., Meyerhans, A., Roclants, G. et Wain-Hobson, S. (1990). Genetic organization of a chimpanzee lentivirus related to VIH-1 (Organisation génétique d'un lentivirus de chimpanzé apparenté au VIH-1). 345, 356-359.
Javaherian, K, Langlois, A. J., LaRosa, G. J., Profy, A. T., Bolognesi, D. P., Herlihy, W. C., Putney, S. D. et Matthews, T. J. (1990). Broadly neutralizing antibodies elicited by the hypervariable neutralizing déterminant of VIH-1 (Anticorps neutralisant un spectre large induit par le déterminant neutralisant hypervariable de VIH-1). Science. 250, 1590-1593.
Javaherian, K, Langlois, A. J., Mc Danal, C., Ross, K L., Eckler. L. I., Jellis, C. L., Profy, A. T., Rusche, J. R., Bolognesi, D. P., Putney, S. D. et Matthews, T. J. (1989). Principal neutralizing domain of the human immunodeficiency virus type 1 envelope protein (Domaine neutralisant principal de la protéine d'enveloppe du virus de l'immunodéficience humaine de type 1). Proc. Natl. Acad. Sci. USA. 86, 6768-6772.
Louwagie, J., McCutchan, F., Peeters, M., Brennan, T., Sanders-Buell, E., Eddy, G., van der Groen, G., Fransen, K, Gershy-Damet, G. et Deleys, R. (1993). Phylogenetic analysis of gag genes from 70 international VIH-1 isolates provides evidence for multiple genotypes (L'analyse phylogénétique de gènes gag de 70 isolats internationaux de VIH-1 fournit la preuve de génotypes multiples). AIDS. 7, 769-80.
Louwagie, J., McCutchan, F., Van der Groen, G., Peeters, M., Fransen, K, Piot, P., Gershy-Damet, G., Roelants, G., Van Heuverswyn, II. et Eddy, G. (1992). Genetic comparison of VIH-1 isolates from Africa, Europe, and North America (Comparaison génétique d'isolats de VIH-1 de l'Afrique, de l'Europe et de l'Amerique du Nord). AIDS Res Hum Retroviruses. 8, 1467-9.
Matsushita, S. M., Robert-Guroff, M., Rusche, J., Koito, A, Hattori, T., Hoshino, H., Javaherian, K, Takatsuki, K et Putney, S. (1988). Characterization of a Human immunodeficiency virus neutralizing monoclonal antibody and mapping of the neutralizing epitope. (Caractérisation d'un anticorps monoclonal neutralisant le virus de l'immunodéficience humaine et cartographie de l'épitope neutralisant). J. Virol 62, 2107-2114.
NKENGASONG, J.N. et al., AIDS 1993, Vol. 7, N° 11, pp. 1536-1538.
Rey, M. A., Krust, B., Laurent, A. G., Montagnier, L. et Hovanessian, A. G. (1989). Characterization of human immunodeficiency virus type 2 envelope glycoproteins : dimerization of the glycoprotein precursor during processing (Caractérisation des glycoprotéines d'enveloppe du virus de l'immunodéficience du type 2 : dimérisation du précurseur glycoprotéique au cours de la maturation). J. Virol. 63, 647-658.
ULMER J.B. et al., Science Vol. 259, Mars 1993, pp. 1745-1749.
Vanden Heasevelde, M., Decourt. J. L., de Leys, R. J., Vanderborght, B., van der Groen, G., van Heuverswijn, H. et Saman, E. (1994). Genomic cloning and complete séquence analysis of a highly divergent African Human Immunodeficiency Virus isolate (Clonage génomique et analyse de la séquence complète d'un isolat africain très divergeant du virus de l'immunodéficience humaine). J Virol. 68, 1566-1596.
Vartanian, J.-P., Meyerhans, A., Asjö, B. et Wain-Hobson, S. (1991). Selection, recombination, and G→A hypermutation of VIH-1 genomes (Sélection, recombinaison, et hypermutation G→A de génomes de VIH-1). J. Virol. 65, 1779-1788.
Wain-Hobson, S., Sonigo, P., Danos, O., Cole, S. et Alizon, M. (1985). Nucleotide séquence of the AIDS virus, LAV (Séquence nucléotidique du virus du SIDA, LAV). Cell 40, 9-17.

## Revendications

1. Acide nucléique permettant la détection du virus HIV-1_{VAU}, comprenant la séquence telle que représentée à la figure 6.

2. Acide nucléique, utilisable comme sonde, consistant en un fragment d'au moins 100 paires de base de la séquence telle que représentée à la Figure 6 d'un acide nucléique selon la revendication 1.

3. Acide nucléique, utilisable comme sonde, **caractérisé en ce qu'**il s'agit d'ADN et consistant en un fragment d'un acide nucléique selon la revendication 1, et qui code pour un polypeptide de la protéine d'enveloppe choisi dans le groupe constitué de séquences qui contiennent ou correspondent à :
(i) la séquence CKNRLIC ;
(ii) la séquence RLLALETFIQNQQLLNLWGCKNRLlC ;
(iii) la séquence RARLLALETFIQNQQLLNLWGCKNRLICYTSVKWNKT ;
(iv) la boucle V3 de séquence CERPGNQTIQKIMAGPMAWYSMALSNTKGDTRAAYC ;
(v) la séquence TFIQN ;
(vi) la séquence WGCKNR ;
(vii) les acides aminés 1 à 526 de la séquence telle que représentée à la Figure 3 ; et
(viii) les acides aminés 527 à 877 de la séquence telle que représentée à la Figure 3.

4. Acide nucléique, utilisable comme sonde, selon l'une quelconque des revendications 1 à 3, **caractérisé en ce qu'**il s'agit d'ADN ou d'ADNc marqué, notamment par des marqueurs radioactifs, enzymatiques ou fluorescents.

5. Acide nucléique selon la revendication 1, **caractérisé en ce qu'**il s'agit d'ARN.

6. Composition pour la détection de la présence ou non d'un rétrovirus HIV-1_{VAU}, dans des échantillons de sérum ou d'autres liquides ou tissus biologiques obtenus à partir de patients susceptibles d'être porteurs d'un rétrovirus HIV-1_{VAU}, ladite composition étant **caractérisée en ce qu'**elle comprend au moins une sonde selon l'une quelconque des revendications 2 à 4.

7. Peptide ou polypeptide choisi dans le groupe constitué de :
- un polypeptide consistant en la séquence telle que représentée à la Figure 3, et codé par un acide nucléique consistant en la séquence telle que représentée à la Figure 6,
- une partie de la protéine env de la séquence telle que représentée à la Figure 3 codée par un acide nucléique consistant en un fragment d'au moins 100 paires de base de la séquence telle que représentée à la Figure 6, et
- un polypeptide (i) de séquence CKNRLIC, (ii) de séquence RLLALETFIQNQQLLNLWGCKNRLIC, (iii) de séquence RARLLALETFI QNQQLLNLWGCKNRLICYTSVKWNKT, (iv) de séquence CERPGNQKI MAGPMAWYSMALSNTKGDTRAAYC, (v) de séquence TFIQN, (vi) de séquence WGCKNR, (vii) consistant en les acides aminés 1 à 526 de la séquence telle que représentée à la Figure 3, ou (viii) consistant en les acides aminés 527 à 877 de la séquence telle que représentée à la Figure 3.

8. Protéine d'enveloppe du rétrovirus HIV-1_{VAU}, **caractérisée en ce qu'**elle peut être obtenue par l'expression dans un hôte cellulaire d'un acide nucléique correspondant à la séquence telle que représentée à la Figure 6, et **en ce que** ladite protéine comprend soit la séquence d'acides aminés comprise entre les résidus 1 et 526 de la séquence telle que représentée à la Figure 3, soit la séquence d'acides aminés comprise entre les résidus 527 à 877 de la séquence telle que représentée à la Figure 3.

9. Polypeptide ou peptide dont la séquence est contenue dans celle des protéines d'enveloppe HIV-1_{VAU} selon la revendication 8, **caractérisé en ce qu'**il comprend un épitope susceptible d'être reconnu par des anticorps induits par le virus HIV-1_{VAU}.

10. Polypeptide ou peptide selon la revendication 9, **caractérisé en ce qu'**il comprend une séquence choisie parmi les séquences suivantes :
(i) la séquence CKNRLIC ;
(ii) la séquence RLLALETFIQNQQLLNLWGCKNRLIC, ou
(iii) la séquence RARLLALETFIQNQQLLNLWGCKNRLICYTSVKWNKT.

11. Polypeptide dérivé du polypeptide selon la revendication 10 (ii) par substitution conservative des acides aminés, dès lors que ledit polypeptide conserve les mêmes caractéristiques antigéniques, choisi parmi le groupe constitué de :
(i) la séquence RLLALETFIQNWWLLNLWGCKNRLIC ; et
(ii) la séquence RLLALETLLQNQQLLSLWGCKGKLVC.

12. Peptide ou polypeptide selon l'une quelconque des revendications 9 à 11, **caractérisé en ce qu'**il s'agit d'un peptide synthétique.

13. Composition appropriée pour la détection *in vitro* de la présence dans un échantillon biologique humain d'anticorps anti-HIV-1_{VAU}, ladite composition comprenant au moins un antigène comprenant un polypeptide ou un peptide de la protéine d'enveloppe d'un rétrovirus HIV-1_{VAU}, tel que défini dans l'une quelconque des revendications 7 à 12.

14. Composition selon la revendication 13, **caractérisée en ce qu'**elle comprend en outre un antigène tel qu'une protéine, une glycoprotéine, un polypeptide ou un peptide d'un virus HIV-1 n'appartenant pas au groupe O et/ou d'un virus HIV-2 ou un peptide dérivé d'un virus HIV-1 n'appartenant pas au groupe O et/ou d'un virus HIV-2 ayant un épitope susceptible d'être reconnu par les anticorps induits par le virus HIV-1 n'appartenant pas au groupe O et/ou le virus HIV-2.

15. Composition selon la revendication 14, **caractérisée en ce que** les protéines et/ou glycoprotéines de HIV-1 n'appartenant pas au groupe O et/ou de HIV-2 sont des protéines GAG, POL ou des fragments des protéines GAG ou POL.

16. Composition selon la revendication 14, **caractérisée en ce que** les protéines et/ou glycoprotéines de HIV-1 n'appartenant pas au groupe O et/ou de HIV-2 sont des glycoprotéines d'enveloppe.

17. Composition selon l'une quelconque des revendications 13 à 16, appropriée pour la détection *in vitro* de la présence dans un échantillon biologique humain d'anticorps anti-HIV-1_{VAU}, **caractérisée en ce que** ladite composition comprend une séquence peptidique correspondant à l'ensemble de la région 590-620 de la protéine gp41 de HIV-1_{VAU} ou à une partie de cette région.

18. Composition selon la revendication 17, **caractérisée en ce que** ladite séquence peptidique est la séquence TFIQN, CKNRLIC ou WGCKNR.

19. Anticorps susceptible de reconnaître une protéine, un peptide ou un polypeptide selon l'une des revendications 7 à 12.

20. Procédé de diagnostic *in vitro* d'une infection causée par le virus HIV-1_{VAU}, ledit procédé comprenant :
- la mise en contact d'un sérum ou d'un autre milieu biologique provenant d'un malade faisant l'objet du diagnostic avec au moins une protéine, un peptide ou un polypeptide selon l'une quelconque des revendications 7 à 12 ou une composition selon l'une quelconque des revendications 13 à 18; et
- la détection d'une réaction immunologique.

21. Réactif nécessaire à la réaction de Western blot ou d'ELISA comportant une protéine, un peptide ou un polypeptide selon l'une des revendications 7 à 12 ou une composition selon l'une quelconque des revendications 13 à 18.

22. Séquence nucléotidique selon la revendication 1 pour utilisation dans l'induction *in vivo* de la synthèse d'anticorps dirigés contre l'antigène codé par ladite séquence.

23. Composition selon l'une quelconque des revendications 13 à 18, capable d'induire des anticorps chez l'animal.

24. Trousse (kit) de détection *in vitro* sur un échantillon biologique, d'un rétrovirus HIV-1_{VAU}, **caractérisée en ce qu'**elle comprend comme sonde éventuellement marquée, au moins une séquence nucléotidique selon l'une quelconque des revendications 1 à 4.

25. Trousse selon la revendication 24, **caractérisée en ce qu'**elle comprend également les réactifs nécessaires à la mise en oeuvre d'une hybridation.

26. Procédé de détection de la présence ou non du virus VIH-1_{VAU} dans des échantillons de sérum ou autres liquides ou tissus biologiques obtenus à partir de patients suspectés d'être porteurs du virus VIH-1_{VAU} comprenant :
- la mise en contact d'une sonde selon l'une quelconque des revendications 2 à 4 soit avec les acides nucléiques obtenus à partir de cellules contenues dans des fluides biologiques, soit avec ces fluides eux-mêmes dès lors que leurs acides nucléiques ont été rendus accessibles à l'hybridation avec cette sonde, et ce dans des conditions permettant l'hybridation entre cette sonde et ces acides nucléiques, et
- la détection de l'hybridation éventuellement produite.

27. Procédé de production d'un antigène, polypeptide ou peptide par expression d'un acide nucléique selon l'une quelconque des revendications 1 à 4.

## Claims

1. A nucleic acid allowing the detection of HIV-1_{VAU} virus, comprising the sequence as shown in Figure 6.

2. A nucleic acid, usable as a probe, consisting of a fragment at least 100 base pairs long of the sequence as shown in Figure 6 of a nucleic acid according to claim 1.

3. A nucleic acid, usable as a probe, **characterized in that** it is DNA and consisting of a fragment of a nucleic acid according to claim 1 which codes for a polypeptide of the envelope protein, selected from the group consisting of the sequences which contain or correspond to:
(i) the CKNRLIC sequence;
(ii) the RLLALETFIQNQQLLNLWGCKNRLIC sequence;
(iii) the RARLLALETFIQNQQLLNLWGCKNRLICYTSVKWNKT sequence;
(iv) the V3 loop with the sequence CERPGNQTIQKIMAGPMAWYSMALSNTKGDTRAAYC;
(v) the TFIQN sequence;
(vi) the WGCKNR sequence;
(vii) amino acids 1 to 526 of the sequence as shown in Figure 3; and
(viii) amino acids 527 to 877 of the sequence as shown in Figure 3.

4. A nucleic acid, usable as a probe, according to any one of claims 1 to 3, **characterized in that** it is DNA or cDNA labeled, in particular by radioactive, enzymatic or fluorescent markers.

5. A nucleic acid according to claim 1, **characterized in that** it is an RNA.

6. A composition for detecting the presence or absence of a HIV-1_{VAU} retrovirus in samples of serum or other biological liquids or tissues obtained from patients susceptible of being carriers of a HIV-1_{VAU} retrovirus, said composition being **characterized in that** it comprises at least one probe according to any one of claims 2 to 4.

7. A peptide or polypeptide selected from the group consisting of:
• a polypeptide consisting of the sequence as shown in Figure 3 and coded by a nucleic acid consisting of the sequence as shown in Figure 6;
• part of the env protein of the sequence coded by a nucleic acid consisting of a fragment of at least 100 base pairs from the sequence as shown in Figure 6; and
• a polypeptide (i) with CKNRLIC sequence, (ii) with RLLALETFIQNQQLLNLWGCKNRLIC sequence; (iii) with the RARLLALETFIQNQQLLNLWGCKNRLICYTSVKWNKT sequence; (iv) with the CERPGNQKIMAGPMAWYSMALSNTKGDTRAAYC sequence; (v) with the TFIQN sequence, (vi) with the WGCKNR sequence, (vii) consisting of amino acids 1 to 526 of the sequence as shown in Figure 3 or (viii) consisting of amino acids 527 to 877 of the sequence as shown in Figure 3.

8. An envelope protein of the HIV-1_{VAU} retrovirus, **characterized in that** it may be obtained by expression in a host cell of a nucleic acid corresponding to the sequence as shown in Figure 6 and **in that** said protein comprises either the amino acid sequence from residues 1 to 526 of the sequence as shown in Figure 3, or the amino acid sequence from residues 527 to 877 shown in the sequence as shown in Figure 3.

9. A polypeptide or peptide the sequence of which is contained in that of the HIV-1_{VAU} envelope proteins of claim 8, **characterized in that** it comprises an epitope which can be recognized by antibodies induced by the HIV-1_{VAU} virus.

10. A polypeptide or peptide according to claim 9, **characterized in that** it comprises a sequence selected from the following sequences:
(i) the CKNRLIC sequence;
(ii) the RLLALETFIQNQQLLNLWGCKNRLIC sequence; or
(iii) the RARLLALETFIQNQQLLNLWGCKNRLICYTSVKWNKT sequence.

11. A polypeptide derived from the polypeptide according to claim 10 (ii) by conservative substitution of amino acids, provided that said polypeptide conserves the same antigenic characteristics, selected from the group consisting of:
(i) the RLLALETFIQNQQLLNLWGCKNRLIC sequence; and
(ii) the RLLALETLLQNQQLLSLWGCKGKLVC sequence.

12. A peptide or polypeptide according to any one of claims 9 to 11, **characterized in that** it is a synthetic peptide.

13. A composition suitable for *in vitro* detection of the presence of anti-HIV-1_{VAU} antibodies in a human biological sample, said composition comprising at least one antigen comprising a polypeptide or a peptide of the envelope protein of a HIV-1_{VAU} retrovirus, as defined in any one of claims 7 to 12.

14. A composition according to claim 13, **characterized in that** it further comprises an antigen such as a protein, a glycoprotein, a polypeptide or a peptide of a HIV-1 virus not belonging to group O and/or of a HIV-2 virus or a peptide derived from a HIV-1 virus not belonging to group O and/or of a HIV-2 virus having an epitope which is capable of being recognized by the antibodies induced by the HIV-1 virus not belonging to group O and/or the virus HIV-2.

15. A composition according to claim 14, **characterized in that** the proteins and/or glycoproteins of HIV-1 not belonging to group O and/or of HIV-2 are GAG, POL proteins or fragments of GAG or POL proteins.

16. A composition according to claim 14, **characterized in that** the proteins and/or glycoproteins of HIV-1 not belonging to group O and/or of HIV-2 are envelope glycoproteins.

17. A composition according to any one of claims 13 to 16, suitable for *in vitro* detection of the presence of anti-HIV- 1_{VAU} antibodies in a human biological sample, **characterized in that** said composition comprises a peptide sequence corresponding to the whole of the 590-620 region of the gp41 protein of HIV-1_{VAU} or to a portion of said region.

18. A composition according to claim 17, **characterized in that** said peptide sequence is the TFIQN, CKNRLIC or WGCKNR sequence.

19. An antibody which is capable of recognizing a protein, a peptide or a polypeptide according to one of claims 7 to 12.

20. A method for *in vitro* diagnosis of an infection caused by the HIV-1_{VAU} virus, said method comprising:
• bringing serum, or another biological medium from a patient to be diagnosed, into contact with at least one protein, peptide or polypeptide according to any one of claims 7 to 12 or a composition according to any one of claims 13 to 18; and
• detecting an immunological reaction.

21. A reagent necessary for Western blot or ELISA reaction, comprising a protein, a peptide or a polypeptide according to one of claims 7 to 12, or a composition according to any one of claims 13 to 18.

22. A nucleotide sequence according to claim 1 for use in *in vivo* induction of the synthesis of antibodies directed against the antigen coded by said sequence.

23. A composition according to any one of claims 13 to 18, capable of inducing antibodies in an animal.

24. A kit for *in vitro* detection of a HIV-1_{VAU} retrovirus in a biological sample, **characterized in that** it comprises, as a probe, optionally labeled, at least one nucleotide sequence according to any one of claims 1 to 4.

25. A kit according to claim 24, **characterized in that** it also comprises the reagents necessary for carrying out hybridization.

26. A method for detecting the presence or absence of HIV-1_{VAU} virus in samples of serum or other biological liquids or tissues obtained from patients suspected of being carriers of HIV-1_{VAU} virus, comprising:
• bringing a probe according to any one of claims 2 to 4 into contact either with nucleic acids obtained from cells contained in biological fluids, or with the fluids themselves provided that their nucleic acids have been rendered accessible to hybridization with said probe, and under conditions allowing hybridization between said probe and said nucleic acids; and
• detecting the hybridization, possibly produced.

27. A method for producing an antigen, polypeptide or peptide by expression of a nucleic acid according to any one of claims 1 to 4.

## Patentansprüche

1. Nucleinsäure, die den Nachweis des HIV-1_{VAU}-Virus ermöglicht, und die die Sequenz wie in Figur 6 dargestellt, umfasst.

2. Als Sonde verwendbare Nucleinsäure, die aus einem Fragment von mindestens 100 Basenpaaren der Sequenz wie in Figur 6 dargestellt besteht.

3. Als Sonde verwendbare Nucleinsäure, die darduch **gekennzeichnet** ist, dass es sich um DNA handelt und dass es aus einem Fragment einer Nucleinsäure nach Anspruch 1 besteht und dass es ein Polypeptid des Hüllproteins codiert, ausgewählt aus der Gruppe bestehend aus Sequenzen, die enthalten oder entsprechen:
(i) Sequenz CKNRLIC;
(ii) Sequenz RLLALETFIQNQQLLNLWGCKNRLIC;
(iii) Sequenz RARLLALETFIQNQQLLNLWGCKNRLICYTSVKWNKT;
(iv) Schleife V3 der Sequenz CERPGNQTIQKIMAGPMAWYSMALSNTKGDTRAAYC;
(v) Sequenz TFIQN;
(vi) Sequenz WGCKNR;
(vii) Aminosäuren 1 bis 526 der Sequenz wie in Figur 3 dargestellt; und
(viii) Aminosäuren 527 bis 877 der Sequenz wie in Figur 3 dargestellt.

4. Als Sonde verwendbare Nucleinsäure nach einem der Ansprüche 1 bis 3, **dadurch gekennzeichnet, dass** es sich um markierte DNA oder RNA handelt, insbesondere mittels radioaktiver, enzymatischer oder fluoreszierender Marker.

5. Nucleinsäure nach Anspruch 1, die **dadurch gekennzeichnet ist, dass** es sich um RNA handelt.

6. Zusammensetzung zum Nachweis der Anwesenheit oder Abwesenheit eines HIV-1_{VAU}-Retrovirus in Serumproben oder anderen biologischen Flüssigkeiten oder Geweben, die von Patienten erhalten wurden, die mögliche Träger eines HIV-1_{VAU}-Retrovirus sind, wobei die Zusammensetzung **dadurch gekennzeichnet ist, dass** sie mindestens eine Sonde nach einem der Ansprüche 2 bis 4 umfasst.

7. Peptid oder Polypeptid, ausgewählt aus der Gruppe bestehend aus:
- einem Polypeptid, das aus der Sequenz wie in Figur 3 dargestellt, besteht und von einer Nucleinsäure codiert wird, die aus der Sequenz wie in Figur 6 dargestellt, besteht,
- einem Teil des env-Proteins der Sequenz der von einer Nucleinsäure codiert wird, die aus einem Fragment von mindestens 100 Basenpaaren der Sequenz wie in Figur 6 dargestellt, besteht, und
- einem Polypeptid (i) der Sequenz CKNRLIC, (ii) der Sequenz RLLALETFIQNQQLLNLWGCKNRLIC, (iii) der Sequenz RARLLALETFI QNQQLLNLWGCKNRLICYTSVKWNKT, (iv) der Sequenz CERPGNQ KIMAGPMAWYSMALSNTKGDTRAAYC, (v) der Sequenz TFIQN, (vi) der Sequenz WGCKNR, (vii) bestehend aus den Aminosäuren 1 bis 526 der Sequenz wie in Figur 3 dargestellt, oder (viii) bestehend aus den Aminosäuren 527 bis 877 der Sequenz wie in Figur 3 dargestellt.

8. Hüllprotein des HIV-1_{VAU}-Retrovirus, **dadurch gekennzeichnet, dass** es erhältlich ist durch die Expression einer Nucleinsäure, die die Sequenz wie in Figur 6 dargestellt, entspricht, in einer Wirtszelle und **dadurch**, dass das Protein entweder die zwischen den Resten 1 und 526 der Sequenz wie in Figur 3 dargestellt, enthaltene Aminosäuresequenz oder die zwischen den Resten 527 bis 877 der Sequenz wie in Figur 3 dargestellt, enthaltene Aminosäuresequenz umfasst.

9. Polypeptid oder Peptid, dessen Sequenz in der Sequenz der HIV-1_{VAU}-Hüllproteine nach Anspruch 8 enthalten ist, **dadurch gekennzeichnet, dass** es ein Epitop umfasst, das von durch das HIV-1_{VAU}-Virus induzierten Antikörpern erkannt werden kann.

10. Polypeptid oder Peptid nach Anspruch 9, **dadurch gekennzeichnet, dass** es eine Sequenz umfasst, die aus den folgenden Sequenzen ausgewählt ist:
(i) Sequenz CKNRLIC;
(ii) Sequenz RLLALETFIQNQQLLNLWGCKNRLIC; oder
(iii) Sequenz RARLLALETFIQNQQLLNLWGCKNRLICYTSVKWNKT.

11. Polypeptid abgeleitet aus dem Polypeptid nach Anspruch 10 (ii) durch konservative Substitution von Aminosäuren, wobei das Polypeptid dieselben antigenen Eigenschaften bewahrt, ausgewählt aus der Gruppe bestehend aus:
(i) der Sequenz RLLALETFIQNWWLLNLWGCKNRLIC; und
(ii) der Sequenz RLLALETLLQNQQLLSLWGCKGKLVC.

12. Peptid oder Polypeptid nach einem der Ansprüche 9 bis 11, **dadurch gekennzeichnet, dass** es sich um ein synthetisches Peptid handelt.

13. Zusammensetzung, die für den In-vitro-Nachweis für die Anwesenheit von anti-HIV-1_{VAU}-Antikörpern in einer biologischen Probe, die vom Menschen stammt, geeignet ist, wobei die Zusammensetzung mindestens ein Antigen umfasst, das ein Polypeptid oder ein Peptid des Hüllproteins eines HIV-1_{VAU}-Retrovirus umfasst, wie in einem der Ansprüche 7 bis 12 definiert.

14. Zusammensetzung nach Anspruch 13, **dadurch gekennzeichnet**, das sie zusätzlich ein Antigen umfasst wie ein Protein, ein Glycoprotein, ein Polypeptid oder ein Peptid eines nicht zur Gruppe O gehörigen HIV-1-Virus und/oder eines HIV-2-Virus oder ein Peptid abgeleitet von einem nicht zur Gruppe O gehörigen HIV-1-Virus und/oder von einem HIV-2-Virus mit einem Epitop, das von den Antikörpern, die durch das nicht zur Gruppe O gehörige HIV-1-Virus und/oder das HIV-2-Virus induziert werden, erkannt werden kann.

15. Zusammensetzung nach Anspruch 14, **dadurch gekennzeichnet, dass** die Proteine und/oder Glycoproteine des nicht zur Gruppe O gehörigen HIV-1 und/oder des HIV-2 GAG- oder POL-Proteine oder Fragmente von GAG- oder POL-Proteinen sind.

16. Zusammensetzung nach Anspruch 14, **dadurch gekennzeichnet, dass** die Proteine und/oder Glycoproteine des nicht zur Gruppe O gehörigen HIV-1 und/oder des HIV-2 Hüllglycoproteine sind.

17. Zusammensetzung nach einem der Ansprüche 13 bis 16, die für den *In-vitro-*Nachweis der Anwesenheit von anti-HIV-1_{VAU}-Antikörpern in einer biologischen Probe, die vom Menschen stammt, geeignet ist, **dadurch gekennzeichnet, dass** die Zusammensetzung eine Peptidsequenz umfasst, die der Gesamtheit oder einem Teil der Region 590-620 des Proteins gp41 von HIV-1_{VAU} entspricht.

18. Zusammensetzung nach Anspruch 17, **dadurch gekennzeichnet, dass** die Peptidsequenz die Sequenz TFIQN, CKNRLIC oder WGCKNR ist.

19. Antikörper, der geeignet ist, ein Protein, ein Peptid oder ein Polypeptid nach einem der Ansprüche 7 bis 12 zu erkennen.

20. In-vitro-Diagnoseverfahren für eine durch das HIV-1_{VAU}-Virus ausgelöste Infektion, wobei das Verfahren umfasst:
- Inkontaktbringen eines Serums oder eines anderen biologischen Mediums, das von einem Kranken stammt, der Gegenstand der Diagnose ist, mit mindestens einem Protein, einem Peptid oder einem Polypeptid nach einem der Ansprüche 7 bis 12 oder einer Zusammensetzung nach einem der Ansprüche 13 bis 18, und
- Nachweis einer Immunreaktion.

21. Für die Western-Blot-Analyse oder ELISA-Analyse erforderliches Reagenz, das ein Protein, ein Peptid oder ein Polypeptid nach einem der Ansprüche 7 bis 12 oder eine Zusammensetzung nach einem der Ansprüche 13 bis 18 enthält.

22. Nucleotidsequenz nach Anspruch 1 zur Verwendung der In-vivo-Induktion der Synthese von Antikörpern, die gegen das von der Sequenz codierte Antigen gerichtet sind.

23. Zusammensetzung nach einem der Ansprüche 13 bis 18, die fähig ist, bei einem Tier Antikörper zu induzieren.

24. Kit für den In-vitro-Nachweis eines HIV-1_{VAU}-Retrovirus in einer biologischen Probe, **dadurch gekennzeichnet, dass** er als optional markierte Sonde mindestens eine Nucleotidsequenz nach einem der Ansprüche 1 bis 4, umfasst.

25. Kit nach Anspruch 24, **dadurch gekennzeichnet, dass** er des Weiteren Reagenzien enthält, die für die Durchführung einer Hybridisierung erforderlich sind.

26. Verfahren für den Nachweis der Anwesenheit oder Abwesenheit des HIV-1_{VAU}-Virus in Serumproben oder anderen biologischen Flüssigkeiten oder Geweben, die von Patienten erhalten wurden, die vermutliche Träger des HIV-1_{VAU}-Virus sind, umfassend:
- Inkontaktbringen einer Sonde nach einem der Ansprüche 2 bis 4 entweder mit Nucleinsäuren, die ausgehend von in biologischen Flüssigkeiten enthaltenen Zellen gewonnen wurden, oder mit den Flüssigkeiten selbst, nachdem ihre Nucleinsäuren für die Hybridisierung mithilfe der Sonde verfügbar gemacht wurden, und dies unter Bedingungen, die die Hybridisierung zwischen der Sonde und den Nucleinsäuren erlauben, und
- Nachweis der möglicherweise herbeigeführten Hybridisierung.

27. Verfahren zur Herstellung eines Antigens, Polypeptids oder Peptids durch Expression einer Nucleinsäure nach einem der Ansprüche 1 bis 4.
